(19)

Europäisches Patentamt
European Patent Office
Office européen des brevets

(11) **EP 3 306 317 A1**

(12) **EUROPEAN PATENT APPLICATION**

(43) Date of publication:
**11.04.2018 Bulletin 2018/15**

(51) Int Cl.:
*G01N 33/533* (2006.01)          *G01N 33/58* (2006.01)
*G01N 33/542* (2006.01)

(21) Application number: **16192290.1**

(22) Date of filing: **04.10.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **Université de Bordeaux**
  **33000 Bordeaux (FR)**
• **Institut Polytechnique de Bordeaux**
  **33402 Talence Cedex (FR)**
• **Centre National de la Recherche Scientifique**
  **75794 Paris Cedex 16 (FR)**

(72) Inventors:
• **PERCHERANCIER, Yann**
  **33140 Villenave d'Ornon (FR)**
• **VEYRET, Bernard**
  **33600 Pessac (FR)**
• **RUIGROK, Hermanus**
  **33000 Bordeaux (FR)**
• **ARBAULT, Stéphane**
  **33170 Gradignan (FR)**
• **SOJIC, Neso**
  **33160 Cestas (FR)**

(74) Representative: **Lecca, Patricia S.**
  **Cabinet Lecca**
  **21, rue de Fécamp**
  **75012 Paris (FR)**

(54) **NOVEL REAL-TIME MULTIPLEXED, MULTI-COLOR BIOLUMINESCENCE RESONANCE ENERGY TRANSFER ASSAY, APPARATUS, AND USES THEREOF**

(57)     The present invention relates to a novel real-time multiplexed, multi-color BRET assay and apparatus for studying protein-protein interactions and determining various biological activities in live cells, wherein the said assay is capable of capturing multiple signals simultaneously from a single sample and simultaneously over one or several wells. The real-time multiplexed, multi-color BRET assay is particularly useful for drug-screening, protein interactions, change of conformation of any channel or receptor subunits within live cells in response to potential drug candidates, thereby indicating activation or inhibition of said channel or receptor.

**Figure 1**

EP 3 306 317 A1

**Description**

**FIELD OF THE INVENTION**

The present invention relates to a novel multiplexed, multi-color BRET assay and apparatus for studying multiple protein-protein interactions per well or sample, and determining various biological activities in live cells, simultaneously over one or several wells, and in real time. The real-time multiplexed, multi-color BRET assay according to the present invention is particularly useful for drug-screening, protein interactions, intracellular biological events monitoring, change of conformation of any channel or receptor subunits within live cells in response to potential drug candidates, thereby indicating activation or inhibition of said channel or receptor.

**BACKGROUND OF THE INVENTION**

Luminescence is a phenomenon in which energy is specifically channeled to a molecule to produce an excited state. Return to a lower energy state is accompanied by release of a photon. Luminescence includes fluorescence, phosphorescence, chemo-luminescence and bioluminescence. Bioluminescence is the process by which living organisms emit light that is visible to other organisms. Where the luminescence is bioluminescence, creation of the excited state derives from an enzyme catalyzed reaction. Luminescence can be used in the analysis of biological interactions.

The life of a cell is ruled by the dynamics of its molecular actors. While multiple external stimuli can share a narrow repertoire of signaling molecules, the fine-tuning of protein complex remodeling in terms of space and time can determine the specificity of cellular responses. Accordingly protein-protein interactions and interplay between proteins and other molecules play a key regulatory role in almost every biological process. Such interactions can be correlated, directly or indirectly, with a variety of intracellular events, such as signal transduction, metabolism, cell motility, apoptosis, cell cycle regulation, nuclear morphology, cellular DNA content, microtubule-cytoskeleton stability, and histone phosphorylation. For example, cytosolic and cell surface protein-protein interactions mediated through various channels or receptors play major roles in normal cellular functions and biological responses. In particular, many cytosolic and cell surface protein-protein interactions are involved in disease pathways. In addition, many protein-protein interactions between factors in cellular transcriptional machineries are also valuable drug targets. Protein-protein interactions are also involved, for example, in the assembly of enzyme subunits; in antigen-antibody reactions; in forming the supramolecular structures of ribosomes, filaments, and viruses; in transport; and in the interaction of receptors on a cell with growth factors and hormones. Products of oncogenes can give rise to neoplastic transformation through protein-protein interactions. Thus, many techniques have been developed to identify and characterize these interactions.

These tools range from in vitro binding assays to library-based methods and include genetic methods such as searching for extragenic suppressors or activators. A technique commonly used for assessing protein-protein interaction is based on fluorescence resonance energy transfer (FRET). In this process, one fluorophore (the "donor") transfers its excited-state energy to another fluorophore (the "acceptor") which usually emits fluorescence of a different color. FRET, however, has several limitations. As with any fluorescence technique, photobleaching of the fluorophore and auto fluorescence of the cells/tissue can significantly restrict the usefulness of FRET, and in highly autofluorescent tissues, FRET is essentially unusable. Also, if the tissue is easily damaged by the excitation light, the technique may be unable to give a value for healthy cells. Finally, if the cells/tissues to be tested are photoresponsive FRET may be impractical because as soon as a measurement is taken, the photoresponse may be triggered. To overcome these disadvantages Bioluminescence Resonance Energy Transfer (BRET) has been developed and is in use for multiple applications. BRET is a natural phenomenon that occurs in a variety of coelenterates, including *Aequorea, Obelia, Phialidium,* and *Renilla.* It is based on the transfer of nonradiative energy originating from the luciferase-mediated oxidation of coelenterazine (the donor) to a fluorescent protein (FP) acting as the energy acceptor, which reemits part of the energy as photons. BRET occurs when the donor and acceptor proteins are in close proximity (typically <100 Å) and when the emission spectrum of the donor overlaps sufficiently with the excitation spectrum of the acceptor.

Over the last decade, resonance energy-transfer approaches have offered new opportunities for real-time probing of the activity of an ever-growing list of proteins in live cells. These techniques are based on the non-radiative transfer of energy between an energy donor and a compatible fluorescent energy acceptor. This is a system of choice for monitoring both constitutive and regulated inter- and intra-molecular interactions. BRET has become a popular, broadly-applicable method, particularly useful in molecular pharmacology, especially concerning G protein-coupled receptors (GPCRs).

In common practice, individual cellular processes are examined sequentially in a number of measurements from different samples, in which common 'fiduciary' events exists. Information about the individual processes is then combined to build a broader picture of the signaling network. Such approaches, termed computational multiplexing, have been applied in reconstructing the spatiotemporal relationship of signaling events measured with respect to, for example, the timing of ligand application, changes in membrane potential, or changes in membrane shape. Useful endogenous fiduciary events do not exist for all processes and exogenous events imposed upon the system often perturb the normal dynamics one wishes to investigate. Furthermore, the interdependence of seemingly stochastic events is an interesting feature and by its nature cannot be studied by computation multiplexing of sequential events.

Therefore, being able to study multiple molecular events simultaneously with a single measurement represents a significant step forward in biology and medicine. Spectral imaging, coupled to mathematical processes, is becoming the gold standard for multiplexed imaging of intracellular molecular events using fluorescent techniques. Also, systems coupling multispectral fluorescence imaging with microscopy and flow cytometry are now commercially available. However, until now, the BRET technique was limited to a filter-based approach that hindered its further development. Specifically, the BRET signals emitted by the donor/acceptor couple required two readers equipped with two dedicated optical filters: a first reader with a filter that corresponds to the energy donor and a second reader with a filter corresponding to the energy acceptor. Experiments have moved beyond using a single fluorophore to the incorporation of multiple fluorophores in a single imaging experiment. Traditionally, this has been done by using a carefully selected set of dyes that have non overlapping spectral emissions, as well as optical filters that can be changed or cycled through to reveal different spectral emissions. A simultaneous multiplexing of spectrally resolved BRET assays within the same cells has been previously reported. However this was a filter-based approach. Filter-based techniques are unable to distinguish whether the signal reaching the detector is from BRET or from the bleed through of the donor emissions into the BRET channel. To this end, a study was reported showing how with such dedicated apparatus and the use of dedicated filters, one can achieve two BRET signals simultaneously within the same well measure (Breton B. et al. Biophysical Journal Volume 99 December 2010 4037-4046). However, these authors must have had to seriously compromise on the choice of filters to be used for realizing their demonstration, given the spectral recovery existing between the donor and the acceptor groups of energy. Moreover, their development requires measuring to be done thrice, first measurement for the energy donor, a second for the first acceptor and a third for the second acceptor. Recently, Hamamatsu developed a system FDSS/µCELL that enables the simultaneous reading of all the wells of a plate with the help of a CCD camera but using only on one BRET probe and necessitating the use of filters. Therefore, it cannot achieve a simultaneous measure of several BRET probes from multiple-wells.

The novel assay and the device described in the current invention enables the acquisition of BRET spectral signals in its entirety, over one or several wells or one or more samples in a simultaneous manner as well as the spectral decomposition of the signals acquired in the donor and acceptor energy signals.

This represents an advantage with reference to the systems already present in the market, because the use of spectral decomposition in place of filters enables the measurement of the BRET signals in one reader, irrespective of the number of probes per well.

Moreover, with the overlapping of the different spectral emissions of the donors and acceptors of usable bioluminescent energy, the measurement and analysis of BRET spectrum enables the mathematic separation of the different elements of the signal obtained. Experimentally, this brings a measurement that is more precise and particularly a simultaneous measurement of the totality of all the donor and acceptor emissions.

This technique has applicability for example over multiple channels and receptors within live cells, including Voltage-dependent ion channels, more specifically Transient Receptor Potential (TRP) ion channels. With this invention, the inventors achieved an unprecedented performance in the field of protein-protein interaction imaging in terms of temporal and spatial resolution, speed of detection and analysis, duration of signal stability, signal sensitivity and dynamic range. This novel development will improve the general comprehension of both the spatio-temporal dynamics of protein-protein interactions and the activation patterns of specific signaling pathways.

## SUMMARY OF THE INVENTION

The present invention provides a real-time multiplexed, multi-color BRET assay for detecting and\or monitoring one or more proteins-proteins interactions in live cells in at least one single well or one or more samples or in a multiplexed analysis, wherein the said assay is capable of capturing multiple signals simultaneously from a single sample and simultaneously over one or several wells, wherein the said multiple signals are emitted in parallel and/or in relay by donor-acceptor pairs according to the invention.

The present invention also provides a real-time multiplexed, multi-color BRET assay for determining and\or monitoring the activity and\or activation or inhibition of channels and\or receptors in live cells, wherein the said assay is capable of capturing multiple signals simultaneously from a single sample and simultaneously over several wells, wherein the said multiple signals are emitted in parallel and/or in relay by donor-acceptor pairs according to the invention.

The present invention further provides a real-time multiplexed, multi-color BRET assay for detecting several connected or independent molecular events simultaneously in live cells, wherein the said assay is capable of capturing multiple signals simultaneously from a single sample and simultaneously over several wells, wherein the said multiple signals are emitted in parallel and/or in relay by donor-acceptor pairs according to the invention.

The present invention provides a novel device or apparatus, novel nucleic acids for construction of novel probes for use in the novel real-time multiplexed, multi-color BRET assay.

The present invention finally provides a method of drug-screening or pharmacologic screening for the identification of new inhibitors or activators of protein targets, discrimination of the effect of a chemical compound/physical stimulus over several pharmacological targets simultaneously, study of the kinetic effect of chemical compound/physical stimuli on several, and simultaneous molecular events.

## BRIEF DESCRIPTION OF THE FIGURES

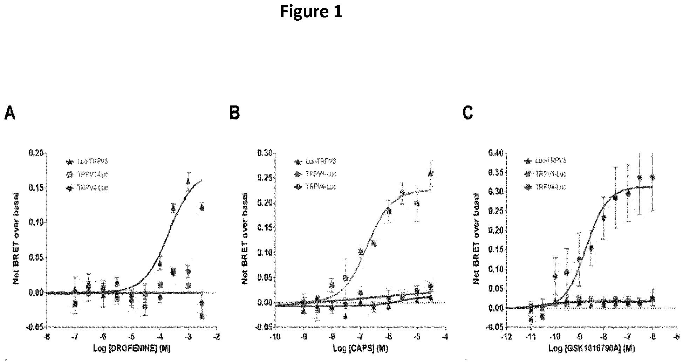

FIGURE 1: Dose-response curve of the effect of Drofenine (A), CAPS (B), and GSK1016790A (C) on HEK293T cells coexpressing YFP-CaM and Luc-TRPV3 (blue), TRPV1-Luc (Green) or TRPV4-Luc (Red). Results are expressed as the difference between net BRET and basal BRET. Under saturating conditions of YFP-CaM expression, basal BRETs of $0.221 \pm 0.004$, $0.202 \pm 0.003$, and $0.275 \pm 0.008$ were measured in HEK293T cells expressing Luc-TRPV3/YFP-CaM, TRPV1-Luc/YFP-CaM, or TRPV4-Luc/YFP-CaM, respectively. As mentioned for TRPV1, these results potentially indicate that TRPV3 and TRPV4 interacted with CaM under non-activated conditions. Results represent the mean $\pm$ SEM of three independent experiments done in duplicate.

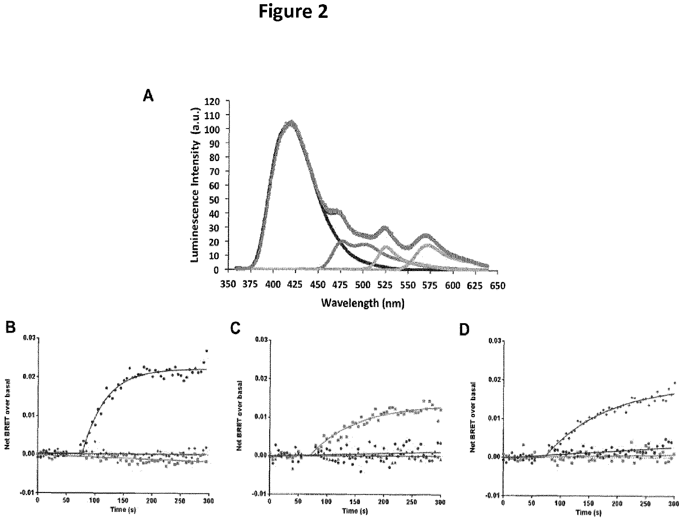

FIGURE 2: Multiplexing measurements of TRPV activity using multicolor BRET. (A) Example of a three-color BRET spectrum and its decomposition, measured in a coculture containing three HEK293T subpopulations transfected with aquamarine-Luc, mAmetrine-Luc, or LSSmOrange-Luc. The grey dots represent the experimental data. The red line is the best fit to the experimental data. Purple, blue, green, and orange lines represent the spectral components of Luc, aquamarine, mAmetrine, and LSSmOrange, respectively. (B to D) Multicolored BRET signals produced by Luc and aquamarine (blue line, basal BRET of $0.027 \pm 0.002$), Luc and mAmetrine (green line, basal BRET of $0.015 \pm 0.001$), and Luc and LSSmOrange (Red line, basal BRET of $0.017 \pm 0.001$) were measured in real time in one sample containing a mixed population of cells expressing Luc-TRPV3/aquamarine-CaM, TRPV1-Luc/mAmetrine-CaM, and TRPV4-Luc/LSSmOrange-CaM constructs. One mM Drofenine (A), 20 $\mu$M CAPS (B), or 100 nM GSK101 (C) were injected 75 seconds after the beginning of the experiment initiated by the injection of purple coelenterazine into the buffer. Results represent the mean of three independent experiments.

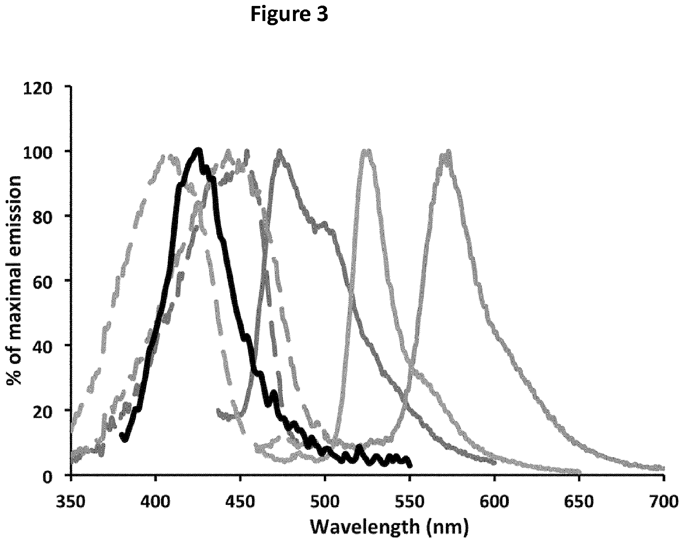

FIGURE 3: Compatibility of the emission spectrum of Luc (in the presence of purple coelenterazine substrate, 2 line from the left), and the absorption (dotted lines) and emission spectra (full lines) of aquamarine ($4^{th}$ dotted line from the left of the graph and $3^{rd}$ full line from the right side of the graph), mAmetrine (first dotted line from the left side of the graph and $2^{nd}$ full line from the right side of the graph), and Lss-mOrange ($3^{rd}$ dotted line from the left side of the graph and last line from the right side of the graph).

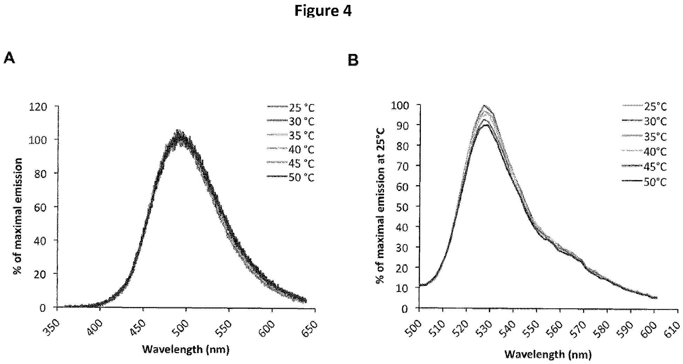

FIGURE 4: shows the emission spectra of Luc (A) and YFP (B) at temperatures ranging from 25 to 50°C.

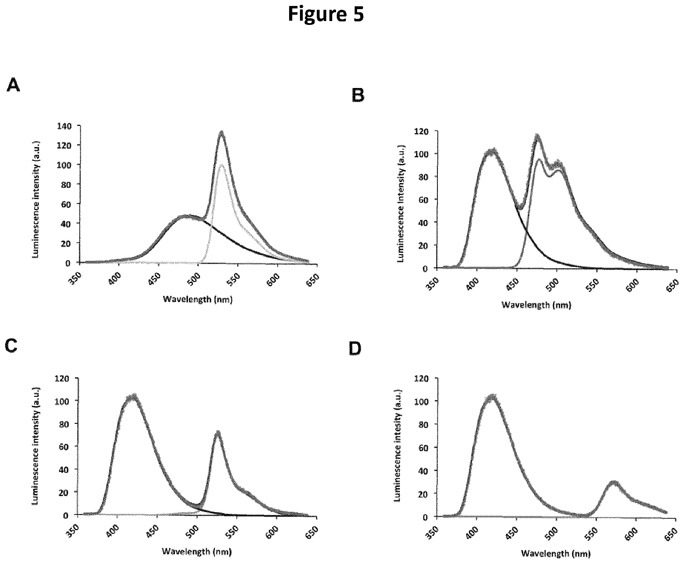

FIGURE 5: Shows the signal decomposition of the bioluminescent spectra measured from HEK293T cell s expressing YFP-Luc (A), aquamarine-Luc (B), mAmetrine-Luc (C), or LSSmOrange -Luc (D). Based on the experimental data (red line), the Lab VIEW interface was used to calculate the shape of the BRET signal and separate the Luc emission spectrum (purple line) from those of YFP (yellow line, A), aquamarine (blue line, B), mAmetrine (green line, C), and LSSmOrange (orange line, 0). The BRET ratio was then calculated by dividing the area under the ,1cceplor spectrum by that under the donor spectrum , thus assuring its independence from any contamination by that of the donor or other acceptors. Net BRET for each FP-Luc is as follow: 0.82 for YFP-Luc, 1.09 for CFP-Luc, 0.43 for mAmetrine-Luc and 0.24 for LssmOrange-Luc. Coelenlerazine H was used as a substrate in A, while purple coelenlerazine was used as a substrate in B-D.

## DETAILED DESCRIPTION OF THE INVENTION

Unless specifically defined otherwise, all technical and scientific terms used herein shall be taken to have the same meaning as commonly understood by one of ordinary skill in the art.

As used herein, the term "real-time" refers to performing a set of operations, such that an output or a result of the set of operations is produced based on a particular timing constraint. While an operation is sometimes referred to herein as being performed in real-time, it is contemplated that an output of the operation can be produced with some detectable delay or latency. For example, an operation can be performed in real-time if an output of the operation is produced at a rate that is the same as or substantially the same as a rate at which an input of the operation is acquired. As another example, an operation can be performed in real-time if an output of the operation is produced within a particular upper limit of response time, such as within 1 second, within 0.1 second, within 0.01 second, or within 0.001 second. As a further example, an operation can be performed in real-time if an output of the operation is timely produced so as to be capable of affecting or controlling a process while it is occurring.

As used herein, the term "multiplex" refers to a BRET assay that provides for simultaneous detection of two or more products or activities within several reaction vessels or reaction wells. Each product or activity is primed using one or more distinct BRET probes.

As used herein the term "multi-color" refers to a BRET assay format of exciting multiple luminescent dyes tagged to one or more probes that produce emission light in relation to target analytes present in the biological sample. The corresponding emission is detected with a multi-color detector or reader which does not require filtering. Light emitted by analytes within a sample or multiple samples can be separated into spectrally distinct components before reaching the image detector to determine relative emission rates from one or more analytes from a single sample or two or more sample constituents having different emission spectra.

As used herein the term "protein-protein interaction" or PPI refers to any kind of interaction, association or binding of two or more proteins together. PPIs may be binary (two protein binding partners; a dimer) or tertiary (three or more protein binding partners, e.g., a trimer). Proteins within a PPI (i.e., binding partners) may be the same protein (such as a homodimer or homotrimer) or different proteins (such as a heterodimer or hetero trimer). Proteins within a tertiary interaction may be bound to one or more proteins within the PPI. This definition extends to all channels and /or receptors/ antigens/antibodies/ cells etc., comprising protein or a protein component.

As used herein, the term "Bioluminescence Resonance Energy Transfer (BRET)" refers to an assay that relies on the energy transfer from a bioluminescent enzyme, a luciferase, and a fluorophore. Unlike FRET (Fluorescence Resonance Energy Transfer), BRET is initiated by an enzymatic reaction and thus does not require light excitation, resulting in an excellent signal to background ratio and greater sensitivity.

"Filter-based BRET assay" refers to an assay wherein the imaging instrumentation is equipped with filter sets for separate and independent measurement of light output at the wavelength corresponding to the emission maximum of the donor and acceptor. The absorption of light results in the formation of excited molecules which can in turn dissipate their energy by decomposition, reaction, or re-emission. The efficiency with which these processes take place is called the quantum efficiency.

As used herein, the term "bioluminescent donor molecule" refers to any molecule able to generate luminescence following either action on a suitable substrate, or its own excitation by an external source. As used herein, the term "acceptor molecule" refers to any compound which can accept energy emitted as a result of the activity of a bioluminescent donor molecule, and re-emit it as light energy.

As used herein "Voltage-dependent ion channels" refer to a group of closely related family of ion channels. Voltage-gated ion channels may be readily identified by function, by structure (both secondary and tertiary), and by sequence homology (primary structure). A hallmark of the voltage-gated ion channels are the six putative transmembrane spanning helices Sl-6 and the "PVP" motif (which is not invariant, e.g., rKv2.1 has a PIP sequence). Within the larger super-family are various families including potassium gated (Kv), sodium gated (Nav), and calcium gated (Cav). The voltage-gated potassium channels fall into a super-family that uses the nomenclature Kv. One family includes four sub-families that were originally named for the four related voltage gated potassium channels from Drosophila: Shaker K i); Shab (Kv2); Shaw (Kv3); and SAa/ (Kv4). Shaker and Shal are characterized as having rapid current activation and inactivation, while Shab and Shaw are delayed rectifier channels that are characterized as having slow inactivation and non-inactivation. Homologues in each sub-family have been identified in humans, rodents, and other mammals. Voltage-dependent ion channels are a proven target for drug discovery, and many ion channel modulators are currently in clinical use for the treatment of pain, epilepsy, hypertension and other disease states. They comprise the molecular basis for essential physiological functions including fluid secretion, electrolyte balance, and bioenergetics and membrane excitability. Ion channels make good drug targets because they are physiologically essential, are pharmacologically accessible, are encoded by a variety of genes and usually operate as multimeric protein assemblies, resulting in a high degree of functional and anatomical specificity. Through molecular cloning, heterologous expression and electrophysiological characterization by patch-clamping, it is clear that the complexity of ion-channel biology also offers multiple opportunities for small-molecule drugs to achieve a specific, desired functional effect. For example, small molecules might influence a variety of biophysical properties of ion channels, such as voltage-dependence permeability, use-dependence, activation and inactivation. In contrast to simple blockers or openers, the discovery of modulatory compounds could allow the development of drugs that specifically act on cells or tissues exhibiting aberrant levels of ion-channel activity. Voltage-dependent ion channels are known to exist in a number of different conformational states, referred to as gating states. For voltage-gated ion channels a channel can be viewed as residing in one of 3 gating states-closed (no ion permeation), open (ion flux occurs) and inactivated (no ion permeation; channel cannot be opened by depolarization), although it should be noted that some channels do not exhibit an inactivated state. Transition between gating states is voltage-dependent, and at any given time, equilibrium exists between these gating states, with the proportion of channels residing in each state depending upon the cellular membrane potential. Many voltage-dependent ion channel modulators have been shown to bind preferentially to a specific gating state or states. For example, the voltage-gated sodium channel blocker lamotrigine is thought to bind to the opened and inactivated states of the brain sodium channel protein. Preferential binding to a particular gating state may occur through an increase in channel affinity for the ion channel modulator, or simply through improved access of the drug to its binding site on the channel.

As used herein, the term "TRP channel" refers to an ion channel protein of the transient receptor potential family of proteins.

As used herein the term "TRP proteins" refers to a group of proteins that form a superfamily of ubiquitously-expressed, functionally-diverse, cation-permeable channels with varying selectivity to several cations. All TRPs are integral proteins containing six transmembrane domains. The N- and C-terminal domains are intracellular and known to be involved in TRP function, regulation, and channel assembly. TRP channels can be activated by several physicochemical means, including the transduction of chemical, temperature, and mechanical stimuli. TRP channels function therefore as polymodal signal integrators that respond by changing their open probability. They are tightly involved into a variety of physiological processes in humans, including sensory physiology, cardiovascular, gastrointestinal, and urological functions, as well as immunity and development. As a result, TRP channel dysfunction has been implicated in many diseases, leading to their emergence as highly promising drug targets (6). Among the TRP channels, six are recognized as thermo-TRPs, expressed in primary somatosensory neurons and activated at specific temperatures. TRPV1-4 transduce elevated temperatures, ranging from moderate (TRPV3 and TRPV4) to noxious heat (TRPV1 and TRPV2), while TRPM8 and TRPA1 are activated by moderate and extreme cold, respectively.

Embodiments of the present disclosure will employ, unless otherwise indicated, techniques of analytical chemistry, synthetic organic chemistry, biochemistry, molecular biology, and the like, which are within the skill of the art. Such techniques are explained fully in the literature. Further the contents of the international publication WO2016131832 are incorporate herein in entirety.

The assay developed according to the present invention cumulatively showed multiplexing capabilities by engaging in multiple-sequential energy transfer steps, either by generating transfers of energy in parallel from more than one molecular probe carrying bioluminescent donor molecule to more than one molecular probe counterpart fluorescent acceptor molecules, or by generating transfers of energy from one molecular probe carrying a bioluminescent donor molecule to more than one molecular probe carrying counterpart fluorescent acceptor molecules or by generating transfers of energy from one molecular probe carrying bioluminescent donor molecule to at least one molecular probe carrying fluorescent acceptor molecule. To create these structures and resulting sequential transfers, donors and acceptors are juxtaposed in a manner in which by sequential activation of energy the donor-acceptor systems according to the invention act in parallel or as a relay and transfers the energy to sequential members either in parallel or in the relay to produce a multi-color spectrum. The emissions are measured over a broad spectral range and donor/acceptor contributions are separated through spectral decomposition. Rather than filtering the signal to maximize the specificity of an emission channel, spectral overlap is used in order to maximize photon collection, with bleed-through negated through linear unmixing. The spectrophotometric analysis of spectral profiles from the resulting parallel or sequential activation processes allow the estimation of the efficiency of each of the transfer steps. The absorption of light or energy results in the formation of excited molecules which can in turn dissipate their energy by decomposition, reaction, or re-emission.

In the assay and method according to the invention, multiple optical signals are generated in response to bio recognition through modulation of the luminescence of populations of donor-acceptor pairs with different emission colours. The donor-acceptor couple interaction that may be used majorly includes bioluminescence resonance energy transfer and fluorescence resonance energy transfer, but can also be applicable towards charge transfer quenching and quenching via proximal gold nanoparticles. Assays for the simultaneous detection of between multiple target analytes have been developed, where spectral decomposition is an important tool. The unique optical properties of the donor-acceptor activation pattern according to the present invention offers several potential advantages in multiplexed detection, and a large degree of versatility, for example, one pot multiplexing at the ensemble level, where only wavelength discrimination is required to differentiate between detection channels. These methods are not being developed to compete with array-based technologies in terms of overall multiplexing capacity, but rather to enable new formats for multiplexed bio analysis. In particular, bio probes based on sequential activation of donor-acceptor interactions are anticipated to provide future opportunities for multiplexed bio sensing within living cells.

The present invention is based on the novel premises of conducting a multiplexed BRET assay using multi-color readouts for simultaneous detection of multiple biological events from a single sample and simultaneously across multiple wells. And such an assay is not based on the traditional filter-based approach rather the present inventors have designed the assay around the decomposition of the whole emission spectrum of the BRET signal. When using multiple probes for reading multiple signals with different filter sets, one faces an inevitable trade-off between the excitation and emission spectra because they are not allowed to overlap: one either sacrifices a large portion of the excitation spectrum (blocking illumination photons and thus sacrificing measurement speed) or acquires a reduced portion of the emission spectrum (blocking emission photons and thus sacrificing signal-to-noise ratio) to accommodate a given filter set. Attempting to use two or three or more BRET probes in a single experiment requires the use of even more highly restricted excitation and emission filter strategies. In most cases assay using two or more probes results in several undesirable consequences, including speed limitations due to the necessity for sequential measurements, reduced sensitivity as the result of smaller filter pass band size, and more complex labeling strategies that are necessary to minimize spectral overlap. Gathering results sequentially requires more time than simultaneous detection and results can be compromised by rapid specimen motions during acquisition. Furthermore, the BRET signal levels in living cells can sometimes be low, especially for specimens with sparse target abundance or those expressing at endogenous levels. Finally, the fluorescent protein color palette is still rather limited and the broad emission profiles make it difficult to cleanly separate emission or else require specialized filter sets. Thus, in live-cell imaging where high speed acquisition is often a mission-critical factor in the success of an experiment, these consequences can have a severe impact on the results of an investigation. To overcome these limitations and explore the potential application of this filter less assay scheme, the current inventors built a BRET assay theme with spectral decomposition of the signals acquired in the donor and acceptor energy signals. This enables the test to be conducted using a single reader. In a preferred embodiment, the present invention provides a real-time multiplexed, multi-color BRET assay wherein the said assay is capable of capturing multiple signals simultaneously from a single sample and simultaneously over several reaction wells, wherein the said assay enables the measurement of the BRET signals in one single reader.

According to the present invention, the real-time multiplexed, multi-color BRET assay may be used for determining and\or monitoring the activity and\or activation or inhibition of any proteins, such as channels and\or receptors in live cells, wherein the said assay is capable of capturing multiple signals simultaneously from a single sample and simultaneously over several reaction wells. Such an assay can be used for measuring multiple biological activities in a living cell, more specifically for detecting several connected or independent molecular events simultaneously in live cells.

Fluorescence Resonance Energy Transfer (FRET) technique, based on intra- and inter-molecular probes, has previously been used to probe conformational changes in various channels in live cells or membranes during activation. Alternatively, FRET has also been applied to studying the interactions of some ion channels with partners, such as Calmodulin. These assays offer the advantage of single-cell microscopy imaging that may be combined with patch-clamp conditions, thus providing a control of channel activation while recording the FRET signal. However a major limitation of the patch clamp technique is its low throughput. Typically, a single, highly trained operator can test fewer than ten compounds per day using the patch clamp technique. Furthermore the technique is not easily amenable to automation, and produces complex results that require extensive analysis. In bioluminescence resonance energy transfer (BRET), the donor fluorophore of FRET is replaced with a luciferase and the acceptor can be any suitable fluorophore.

The use of a luciferase avoids the need for illumination as the addition of a substrate initiates bioluminescent emission and hence resonance energy transfer. Eliminating the need for an external light source for donor excitation gives BRET some advantages over FRET: it does not cause photo damage to cells, photo bleaching of fluorophores, background auto fluorescence, or direct excitation of the acceptor. Thanks to these advantages, the BRET technique has been widely implemented for drug screening, especially in the GPCR research field. The present invention recognizes for the first time a multiplexed multi-color BRET analysis is possible when based on spectral decomposition.

The present invention thus provides a real-time multiplexed, multi-color bioluminescence resonance energy transfer (BRET) technology-based assay for detecting and\or monitoring one or more proteins-proteins interactions simultaneously in live cells and optionally in multiple reaction wells, wherein said live cells are recombinant cells comprising one or more molecular probes carrying bioluminescent donor molecules, and one or more molecular probes carrying at least two fluorescent acceptor molecules, wherein said bioluminescent donor and its corresponding fluorescent acceptor molecules form donor-acceptor couple which are selected such that the emission spectrum of the bioluminescent donor molecule overlaps with the absorbance spectrum of the fluorescent acceptors molecules, thereby (i) generating transfers of energy in parallel from more than one molecular probe carrying bioluminescent donor molecule to more than one molecular probe counterpart fluorescent acceptor molecules, or (ii) generating transfers of energy from one molecular probe carrying a bioluminescent donor molecule to more than one molecular probe carrying counterpart fluorescent acceptor molecules, or (iii) generating transfers of energy from one molecular probe carrying bioluminescent donor molecule to at least one molecular probe carrying fluorescent acceptor molecule, whereby the resulting emission spectrum of said activated fluorescent acceptor molecule overlaps with the absorbance spectrum of a subsequent acceptor molecule, thereby allowing transfer of energy in cascade of subsequent fluorescent acceptor molecules, wherein energy signals of said each donor-acceptor couples are sufficiently distinct so as to allow spectral decomposition, said assay comprising the steps of:

(i) contacting live recombinant cells with an activation or inhibition signal;
(ii) capturing multiple energy signals from each donor-acceptor couple simultaneously from a single sample or well and optionally simultaneously over several samples or wells,
(iii) processing said multiple energy signals by spectral decomposition.

Preferably, the multiple emissions or multiple energy signals are captured across visible spectra close to the infrared spectrum, most preferably including substantially all wavelengths of light from 400 to 800 nm.

Particularly, the assay according to the present invention does not use a filter-based assay format. Rather the assay is dependent on decomposition of the whole emission spectrum of the BRET signal. The applicable principle is the transformation of the spectral information (from spectrophotometer) into an image (via an imaging system) which will provide a mathematical or graphic means of mapping protein-protein interactions. In effect, the assay according to the present invention is based on full spectral multi-color output by virtue of one or more BRET donors and multiple FRET acceptors and their corresponding pairing and interactions that leads to various spectrally decipherable excitation states within the assay.

Preferably, the real-time multiplexed, multi-color BRET assay according to the present invention, enables the measurement of the BRET signals in a single reading and as one output. The ability to multiplex standard assays with BRET through a single reader allows users to extract more information than ever from a single well and across multiple samples in multiple wells.

Bioluminescent donor molecules are well-known in the art, and we can cite bioluminescent donor molecules chosen from among luciferase, chosen among Renilla luciferase, Firefly luciferase, Coelenterate luciferase, North American glow worm luciferase, click beetle luciferase, a railroad worm luciferase, Gaussia luciferase, Aequorin, Arachnocampa luciferase, or a biologically active variant or fragment of any one, or non-luciferase bioluminescent protein chosen among β-galactosidase, lactamase, horseradish peroxydase, alkaline phosphatase, β-glucuronidase, or β-glucosidase.

Fluorescent acceptor molecules are also well-known in the art and may be chosen from among green fluorescent protein (GFP), variant of green fluorescent protein (GFP10), blue fluorescent protein (BFP), cyan fluorescent protein (CFP), yellow fluorescent protein (YFP), enhanced GFP (EGFP), enhanced CFP (ECFP), enhanced YFP (EYFP), GFPS65T, Emerald, Topaz, GFPuv, destabilised EGFP (dEGFP), destabilised ECFP (dECFP), destabilised EYFP (dEYFP), HcRed, t-HcRed, DsRed, DsRed2, mRFP1, pocilloporin, Renilla GFP, Monster GFP, paGFP, Kaede protein or a Phycobiliprotein, or a biologically active variant or fragment of any one thereof, or wherein the acceptor molecule is Alexa, fluor dye, Bodipy dye, Cy dye, fluorescein, dansyl, umbelliferone, fluorescent microsphere, luminescent nanocrystal, Marina blue, Cascade blue, Cascade yellow, Pacific blue, Oregon green, Tetramethylrhodamine, Rhodamine, Texas red, rare earth element chelates, mAmetrine, LSSmOrange, aquamarine or any combination or derivatives thereof.

The real-time multiplexed, multi-color BRET assay according to the present invention provides an assay for a protein of interest, wherein the said assay includes probes comprising nucleic acids encoding the protein or fragments of the protein, wherein one or more bioluminescent donor molecules and fluorescent acceptor molecules are fused to proteins of interest within said molecular probes, thereby allowing monitoring and/or detection of said proteins-proteins interactions. In particularly preferred embodiments the protein of interest is a membrane protein, a cytoplasmic protein, a nuclear protein and the like. Proteins as referred to herein can be a component which is made totally of protein or it could be a component comprising a protein.

The present invention also relates to a device or apparatus for conducting the real-time multiplexed, multi-color BRET assay. The applicable principle is the transformation of the spectral information (from spectrophotometry) into an image (via an imaging system) which will provide a mathematical or graphic means of mapping protein-protein interactions.

The spectrometers according to the current invention can operate with multiple variables that have a significant influence on band pass, wavelength dispersion, aberrations, and light throughput. The invention includes within its scope spectrometers that can be coupled with linear arrays or charge coupled devices (CCD) as a wavelength detectors. Further spectrometers with various wavelength dispersive elements (WDE) are within the scope of the invention. In certain embodiments, wavelength dispersive element (WDE) is a prism or diffraction grafting. In most preferred embodiments the wavelength dispersive element is diffraction grafting. Further spectrophotometer with either a one-dimensional linear array of detector elements, or a matrix array such as a charge coupled devices (CCD) can acquire a series of wavelengths simultaneously. CCD is preferably chosen. Further Diffraction gratings can be chosen from classically ruled (CR), holographic surface relief (HSRG), and volume holographic (VHG). A classical diffraction grating is generated by mechanically "ruling" (actually burnishing) grooves into a coating of aluminum or gold on a glass blank. Holographic gratings are recorded at the intersection of two expanded laser beams to form a series of periodic fringes in photoresist, which, after processing, form sinusoidal grooves. A key advantage to these gratings is that they do not require any additional focusing or collimating optics. Virtually all diffraction gratings diffract light into "orders," with the "first" order used to present spectral data. All wavelengths are diffracted simultaneously; so all orders, which can be present, will be present. Therefore, if 600 nm is diffracted into first order, then 300 nm will be present in second order, 200 nm in third order, and so on. Any and all kinds of hyper spectral or multispectral imaging systems are within the scope of the invention.

Preferably, the device or apparatus for performing real-time multiplexed, multi-color BRET assay, comprises a real time BRET instrument for capturing the spectral emission, a composition or reaction mixture comprising BRET probes and samples, and an imaging device for conversion of spectral data into a readable output. In preferred embodiments the imaging device is adapted for color-imaging detection, which could refer to any component, portion thereof, or system of components that can detect colored light including a charged coupled device (CCD), back-side-thinned, cooled CCD, front-side illuminated CCD, a CCD array, a photodiode, a photodiode array, a photo-multiplier tube (PMT), a PMT array, complimentary metal-oxide semiconductor (CMOS) sensors, CMOS arrays, a charge-injection device (CID), CID arrays, etc. The imaging detector can be adapted to relay information to a data collection device for storage, correlation, and/or manipulation of data, for example, a computer, or other signal processing system.

Most preferably, the present invention provides an apparatus for performing real-time multiplexed, multi-color BRET assay comprising a real-time BRET instrument and multiple reaction wells for containing said live cells, a spectrophotometer with a suitable imaging system, one or more optic fibers, said spectrophotometer with suitable imaging system and optic fibers being connected to a computer equipped with an information interface for the collection and interpretation of the decomposition of the spectral signals acquired and/or for sending back the form and area of the spectra of the energy donor and of the energy acceptors in a quantitative manner. The imaging system may comprise multiple components or a combination of multiple components or features selected from one or more of diffraction grating, hyper spectral imaging, and/or a CCD camera.

Typically, optic fibers allow capturing of the bioluminescent signals produced by the samples. Said optic fibers carry the captured ligths or signals to a spectrophotomoter which comprises a diffraction grafting and a CCD camera. Therefore, the diffracted light spectrum is recorded on the CCD camera, and then processed via mathematical spectral decomposition allowing to extract spectra of the donor and acceptor molecules.

The BRET apparatus or device according to the invention comprises BRET instrument comprising multiple reaction wells for containing reaction mixture, a spectrometer furnished with diffraction grating, a CCD camera, one or more fiber optics, all of this connected to a computer equipped with an information interface that allows for the decomposition of the spectral signals acquired as well as sending back the form and area of the spectra of the energy donor and of the energy acceptors in a quantitative manner.

The detection system may include, but is not limited to, compact module and an imaging device disposed in the module. The imaging device can include, but is not limited to, a CCD camera and a cooled CCD camera. Charged coupled device (CCD) detectors are made of silicon crystals sliced into thin sheets for fabrication into integrated circuits using similar technologies to those used in making computer silicon chips. For a detailed overview of CCD technology, please refer to Spibey et al. (2001, Electrophoresis 22: 829-836).One of the properties of silicon-based detectors is their high sensitivity to light, allowing them to detect light in the visible to near-infrared range. CCD cameras operate by converting light photons at wavelengths between 400 and 1000 nm that strike a CCD pixel with energy of just 2-3 eV into electrons. A CCD contains semiconductors that are connected so that the output of one serves as the input of the next. In this way, an electrical charge pattern, corresponding to the intensity of incoming photons, is read out of the CCD into an output register and amplifier at the edge of the CCD for digitization. Older intensified CCD cameras had much lower sensitivities than newer-generation cooled CCD cameras. This is because thermal noise (termed "dark-current") from thermal energy within the silicon lattice of a CCD chip resulted in constant release of electrons. Thermal noise is dramatically reduced if the chip is cooled; dark current falls by a factor of 10 for every 20° C. decrease in temperature. In the BRET system, the CCD camera is usually mounted in a light-tight specimen chamber, and is attached to a cryogenic refrigeration unit (for camera cooling. A camera controller, linked to a computer system, is used for data acquisition and analysis. The spectral emission is collected by optical fiber linked to a spectrometer like Spectra Pro 2300i or any equivalents, equipped with a liquid-nitrogen-cooled CCD camera for recording the full visible spectrum. The diffraction grating disperses the transmitted light into spatially-separated wavelength components that are received by the image sensor (CCD). Using the LabView programming language (National Instruments, Austin, Tx, USA or any other equivalents, an interface can be developed to run the acquisition of the bioluminescent spectra and perform real-time spectral decomposition of the BRET signal into its various components.

According to one embodiment, the real-time multiplexed, multi-color BRET assay according to the present invention provides an assay for a protein which is a receptor or a voltage-dependent ion channel. Further provided are probes comprising nucleic acids encoding such receptor or a voltage-dependent ion channels or various fragments of the same wherein one or more bioluminescent donor molecules and fluorescent acceptor molecules are fused to the nucleic acids within said molecular probes, thereby allowing monitoring and/or detection of said receptor or a voltage-dependent ion channel.

According to this embodiment, the novel assay and apparatus for real-time BRET may be used for measurement of one or multiple TRPV ion channels, more specifically TRPV1, TRPV3, and TRPV4 ion-channel activation in live cells. A decomposition of the whole emission spectrum of the BRET signal, instead of the usual selective filter-based approach, provided for the first time a reliable method for performing three-color BRET tests. This novel approach was used to observe the selective activation of multiple TRPV ion channels, more specifically TRPV1, TRPV3, and TRPV4 in a single assay, simultaneously, in real time. This is a significant advancement, because implementation of high-throughput screening (HTS) on ion channels, including TRPs, has proved more problematic. The gold standard for evaluating the activity of TRPs and other ion channels is patch-clamp electrophysiology. Although improvements that increase throughput for the direct screening of ion channel targets are rapidly emerging, including automated electrophysiology and planar patch-clamp techniques, these approaches remain expensive and require expert handling. For HTS, indirect readout technologies are often used as an initial screening step, later confirmed by patch-clamp. These techniques usually rely on fluorescent assays to monitor changes in membrane potential or intracytoplasmic calcium concentrations. Nonetheless, indirect assays of ion channel function often produce false-positive hits, as they monitor endpoints distal from the channel, separated by multiple steps in the signaling pathways. Measuring events proximal to receptor activation reduces the probability of false positives. Therefore, the advent of BRET probes for monitoring the activation of channels in live cells in real time is most valuable. While steady-state TRPV1 subunit oligomerization had been previously studied using either FRET or a combination of BiMolecular Fluorescence complementation and BRET, none of these authors could show any variation of the measured signal following TRPV1 activation. Several studies succeeded in measuring ion-channel activation using intra- or intermolecular FRET based probes, but only one research group successfully reported the use of BRET-based biosensors to monitor ion-channel activity, focusing on the Kir3 inwardly-rectifying potassium channel in combination with FlAsH (fluorescein arsenical hairpin binder). Adapting the FlAsH/BRET approach to other channels would require extensive studies to determine how to insert the FlAsH sequence into the channel structure to yield optimal variation in BRET signal upon channel activation. Moreover, the alterations in BRET signal were often weak, not exceeding 5-10% of the basal BRET signal. In sharp contrast, experiments according to the instant invention, using TRPV-Luc/YFP-CaM detected significantly larger increases upon activation, ranging from 65 to 115 % of the corresponding basal net BRET. These probes offer a wide potential for developing simple cell-based assays that provide direct information on channel activity. The present invention recognizes for the first time a non-filter based BRET analysis of voltage regulated ion channels, wherein the said analysis is based on real-time multiplexed, multi-color BRET assay for determining activation or inhibition of voltage regulated ion channels in live cells, wherein the said assay employs 2 or more BRET probes specific for one or more voltage regulated ion channels, and wherein the said assay is capable of capturing multiple signals simultaneously from a single sample and simultaneously over several wells. The present invention also includes instrumentation and methods that provide for the accurate and reliable information generation. An object of the present invention is to provide a screening system that targets ion channels and has superior efficiency. The present invention provides an improved assay and materials for multiplexed screening for compounds that act on a target ion channel. In a specifically preferred embodiment, the present invention provides a non-filter based real-time multiplexed, multi-color BRET assay capable of reading one or multiple wells OR SAMPLE at one time for one or more voltage-dependent ion channel, wherein the said voltage-dependent ion channel is a transient receptor potential (TRP) channel.

The full-spectral BRET multiplexing assay according to the present invention may also be of importance to monitor several molecular events simultaneously or to evaluate the kinetic of their engagement. For example, it is known that a single receptor in the G-protein coupled receptor family engages different signaling pathways and that various drugs binding to this membrane protein may differentially influence each of them, leading to a reassessment of the efficacy concept. In other words, ligands that are agonist for a given signaling pathway may act as antagonist or even inverse agonist for a different pathway via the same receptor. The large network of protein-protein interactions in ion-channel pathways offers a rich source of potential drug targets which can be tapped successfully by employing the novel assay according to the present invention.

TRP channels in general and TRPV1 channel in particular, for example, has been shown to interact with multiple partners, such as Caveolin, β-Arrestin-2, AKAP79/150 and PKCβ2, as well as other TRP channels. Constructing novel BRET probes to test the interactions between TRPV1 and each of these partners greatly contributes to resolving the complex, dynamic interplay between TRPV1 and its interactions, thus offering new effective methods for screening macromolecular complexes in search of new compounds that target protein-channel interfaces. In this context, monitoring multiple signaling pathways via a single multi-color assay protocol represents a highly valuable development.

The invention also provides a useful tool to probe for determining any interactions, conformational changes, etc.. occurring within a protein of interest. As a result, by multiplexing different BRET-biosensors of the protein of interest, the invention offers the possibility to set up pharmacological fingerprints that are specific to each protein, thus allowing differentiating the distinct signaling modes of different ligand toward the various signaling pathways engaged. Specific probes for use in the present invention can be constructed as described in the Examples below.

According to one embodiment, novel probes may comprise the novel nucleic acids encoding voltage-dependent ion channel fusion subunit comprising one or more bioluminescent donor molecule and at least two fluorescent acceptor molecules. According to this embodiment, novel BRET probes may comprise of a nucleotide sequence encoding voltage-dependent ion channel fusion subunit is bound to a nucleotide sequence encoding one bioluminescent donor molecule and/or bound to a nucleotide sequence encoding at least two fluorescent acceptor molecules.

According to the present invention, voltage-dependent ion channel subunit may be voltage-dependent cation channel or voltage-dependent anion channel. Given the complex structure of these voltage-dependent ion channels which can comprise from 2 to 24 transmembrane domains, it was surprising that such a fusion subunit with BRET donor and acceptor tags would retain structural and functional integrity. In a preferred embodiment of the present invention, the voltage-dependent ion channel subunits may comprise subunit of a voltage-dependent anion channel. The voltage-dependent ion channel subunits may comprise subunit of a voltage-dependent cation channel. These channels can be of any source as long as when expressed in a cell, the N-terminus and C-terminus are intracytoplasmic. In certain preferred embodiments, the novel nnucleic acids according to present invention and thereby the probes comprising the same can have any of the following placements wherein (i) the bioluminescent donor molecule is bound to C-terminal of channel subunit and acceptor molecules are bound to N-terminal of said channel subunit, (ii) the bioluminescent donor molecule is bound to N-terminal of channel subunit and acceptor molecules are bound to C-terminal of said channel subunit, (iii) the bioluminescent donor molecule is bound to C-terminal of channel subunit and acceptor molecules forms part of the first or the second intracellular loop, (iv) the bioluminescent donor molecule is bound to N-terminal of channel subunit and acceptor molecules forms part of the first or the second intracellular loop, (v) said acceptor molecules are bound to C-terminal of channel subunit and the bioluminescent donor molecule forms part of the first or second intracellular loop, (vi) said acceptor molecules are bound to N-terminal of channel subunit and the bioluminescent donor molecule forms part of the first or second intracellular loop, (vii) the bioluminescent donor molecule forms part of the first intracellular loop and the acceptor molecules forms part of the second intracellular loop, or (viii) the bioluminescent donor molecule forms part of the second intracellular loop and the acceptor molecules forms part of the first intracellular loop.

Therefore, according to this embodiment, the present invention provides probes for use in real-time multiplexed, multi-color BRET assay comprising a nucleic acid comprising a nucleotide sequence encoding any protein or channel or receptor or more specifically voltage-dependent ion channel fusion subunit comprising a voltage-dependent cation channel subunit bound to at least one bioluminescent donor molecule and bound to at least one fluorescent acceptor molecule, wherein said voltage-dependent cation channel subunit is a subunit of a transient receptor potential (TRP) channel, and wherein said bioluminescent donor molecule and acceptor molecule are selected so that the emission spectrum of the bioluminescent donor molecule overlaps with the absorbance spectrum of the acceptor molecule, so the light energy delivered by the bioluminescent donor molecule is at a wavelength that is able to excite the acceptor molecule. In certain preferred embodiments the said subunit belongs to TRPV1, TRPV3, or TRPV4 channel. In most preferred embodiments the probe is Luc-TRPV/YFP-CaM selected from Luc-TRPV3/YFP-CaM, TRPV1-Luc/YFP-CaM, TRPV4-Luc/YFP-CaM and\or their equivalents.

This invention further relates to a method of identifying a compound or a candidate capable of binding to a target domain of a channel or receptor, more specifically voltage-dependent ion channel by providing a nucleic acid comprising a nucleotide sequence encoding a channel or receptor, more specifically a voltage-dependent ion channel fusion subunit bound to a nucleotide sequence encoding at least one bioluminescent donor molecule and/or bound to a nucleotide sequence encoding at least 2 fluorescent acceptor molecules..

The present invention also relates to an expression vector comprising an acid nucleic encoding a voltage-dependent ion channel fusion subunit as described above. The present invention also relates to a cell, genetically engineered with the nucleic acid or polynucleotide of the present invention or the vector of the present invention. The cell may be in cell culture or part of a host. Said cell or said host may be produced by introducing said polynucleotide or vector(s) into a cell or host which upon its/their presence mediates the expression of the polypeptide (*i.e.,* fusion subunit) encoded by said nucleic acid or polynucleotide.

The cell or host may be any prokaryote or eukaryotic cell. The host may be any prokaryote or eukaryotic cell. The present invention is also directed to a recombinant host cell containing an expression vector for expression of voltage-dependent ion channel subunit, wherein said vector contains a polynucleotide comprising a nucleic acid sequence encoding the voltage-dependent ion channel subunit or a functionally equivalent active fragment thereof as described above.

Recombinant cells according to the present invention thus comprise an expression vector wherein said channel fusion subunit is expressed and is able to co-assemble with other homomeric or heteromeric channel subunits *in vitro* and *in vivo* to form a functional channel. The present invention embodies a process for the production of said channel fusion subunits as described above comprising culturing said recombinant cell according to the invention, and expressing said channel fusion subunit. The present invention also relates to expression vectors comprising nucleotide sequences encoding the voltage-dependent channel fusion subunits, recombinant cells comprising such expression vectors, process for the production of voltage-dependent ion channel subunit, as well as to the voltage-dependent fusion subunits *per se.*

## Assay for BRET

Although non-filter based assay is the most preferred format for the assay according to the current invention, the novel nucleic acids and probes comprising the same can also be used in filter-based assays. Accordingly in a preferred embodiment, the energy transfer occurring between the bioluminescent protein and acceptor molecule is presented as calculated ratios from the emissions measured using optical filters (one for the acceptor molecule emission and the other for the bioluminescent protein emission) that select specific wavelengths (see equation 1).
Equation 1:

$$Ea/Ed = BRET\ ratio\ (1)$$

where Ea is defined as the acceptor molecule emission intensity (emission light is selected using a specific filter adapted for the emission of the acceptor) and Ed is defined as the bioluminescent protein emission intensity (emission light is selected using a specific filter adapted for the emission of the bioluminescent protein).

It should be readily appreciated by those skilled in the art that the optical filters may be any type of filter that permits wavelength discrimination suitable for BRET. For example, optical filters used in accordance with the present invention can be interference filters, long pass filters, short pass filters, etc. Intensities (usually in counts per second (CPS) or relative luminescence units (RLU)) of the wavelengths passing through filters can be quantified using either a photo-multiplier tube (PMT) or a CCD camera. The quantified signals are subsequently used to calculate BRET ratios and represent energy transfer efficiency. The BRET ratio increases with increasing intensity of the acceptor emission.

Generally, a ratio of the acceptor emission intensity over the donor emission intensity is determined (see equation 1), which is a number expressed in arbitrary units that reflects energy transfer efficiency. The ratio increases with an increase of energy transfer efficiency.

Energy transfer efficiencies can also be represented using the inverse ratio of donor emission intensity over acceptor emission intensity (see equation 2). In this case, ratios decrease with increasing energy transfer efficiency. Prior to performing this calculation the emission intensities are corrected for the presence of background light and auto-luminescence of the substrate. This correction is generally made by subtracting the emission intensity, measured at the appropriate wavelength, from a control sample containing the substrate but no bioluminescent protein, acceptor molecule or polypeptide of the invention.
Equation 2:

$$Ed/Ea = BRET\ ratio\ (2)$$

where Ea and Ed are as defined above

The light intensity of the bioluminescent protein and acceptor molecule emission can also be quantified using a monochromator-based instrument such as a spectrofluorometer, a charged coupled device (CCD) camera or a diode array detector. Using a spectrofluorometer, the emission scan is performed such that both bioluminescent protein and acceptor molecule emission peaks are detected upon addition of the substrate. The areas under the peaks represent the relative light intensities and are used to calculate the ratios, as outlined above. Any instrument capable of measuring lights for the bioluminescent protein and acceptor molecule from the same sample can be used to monitor the BRET system of the present invention.

In an alternative embodiment, the acceptor molecule emission alone is suitable for effective detection and/or quantification of BRET. In this case, the energy transfer efficiency is represented using only the acceptor emission intensity. It would be readily apparent to one skilled in the art that in order to measure energy transfer, one can use the acceptor emission intensity without making any ratio calculation. This is due to the fact that ideally the acceptor molecule will emit light only if it absorbs the light transferred from the bioluminescent protein. In this case only one light filter is necessary.

In a related embodiment, the bioluminescent protein emission alone is suitable for effective detection and/or quantification of BRET. In this case, the energy transfer efficiency is calculated using only the bioluminescent protein emission intensity. It would be readily apparent to one skilled in the art that in order to measure energy transfer, one can use the donor emission intensity without making any ratio calculation. This is due to the fact that as the acceptor molecule absorbs the light transferred from the bioluminescent protein there is a corresponding decrease in detectable emission from the bioluminescent protein. In this case only one light filter is necessary.

In an alternative embodiment, the energy transfer efficiency is represented using a ratiometric measurement which only requires one optical filter for the measurement. In this case, light intensity for the donor or the acceptor is determined using the appropriate optical filter and another measurement of the samples is made without the use of any filter (intensity of the open spectrum). In this latter measurement, total light output (for all wavelengths) is quantified. Ratio calculations are then made using either equation 3 or 4. For the equation 3, only the optical filter for the acceptor is required. For the equation 4, only the optical filter for the donor is required.
Equation 3:

$$Ea/Eo\text{-}Ea = BRET\ ratio\ or\ = Eo\text{-}Ea/Ea\ (3)$$

Equation 4:

$$Eo\text{-}Ed/Ed = BRET \text{ ratio or} = Ed/Eo\text{-}Ed \quad (4)$$

where Ea and Ed are as defined above and Eo is defined as the emission intensity for all wavelengths combined (open spectrum).

It should be readily apparent to one skilled in the art that further equations can be derived from equations 1 through 4. For example, one such derivative involves correcting for background light present at the emission wavelength for bioluminescent protein and/or acceptor molecule.

In performing a BRET assay, light emissions can be determined from each well using the BRETCount. The BRETCount instrument is a modified TopCount, wherein the TopCount is a microtiterplate scintillation and luminescence counter sold by Packard Instrument (Meriden, CT). Unlike classical counters which utilise two photomultiplier tubes (PMTs) in coincidence to eliminate background noise, TopCount employs single- PMT technology and time-resolved pulse counting for noise reduction to allow counting in standard opaque microtiterplates. The use of opaque microtiterplates can reduce optical crosstalk to negligible level. TopCount comes in various formats, including 1, 2, 6 and 12 detectors (PMTs) which allow simultaneous reading of 1, 2, 6 or 12 samples, respectively. Beside the BRETCount, other commercially available instruments are capable of performing BRET: the Victor 2 (Wallac, Finland (Perkin Elmer Life Sciences)) and the Fusion (Packard Instrument, Meriden). BRET can be performed using readers that can detect at least the acceptor molecule emission and preferably two wavelengths (for the acceptor molecule and the bioluminescent protein) or more.

In an embodiment of the invention, BRET is detected using a microfluidics device. Microfluidics devices conveniently require only an aliquot of the sample, generally not more than about 50 μL, to be transferred to the sample reservoir of the micro fluidics device. This is performed either manually or by pneumatic injection via a syringe, capillary or the like.

An automated luminescence biochip device using microfluidics may be used to perform all the necessary BRET reaction steps. Automating BRET reactions in a microfluidic biochip platform is desirable as this avoids multiple manual handling steps and reduces human time and effort in performing experiments. The microfluidics device may contain a self-contained disposable biochip with patterned microchannels and compartments having storage means for storing a plurality of samples, reagents, and substrates. The steps of transferring sequentially at least one of the samples, or reagents, and then luminescent substrate from compartments through microchannels to the reaction sites could be automated. The luminescent substrates would then react with the donor molecules resulting in luminescence, which would be detected by an optical detector. An example of a microfluidics device for detecting luminescence is described in US Patent Application No. US 6,949,377.

In a further aspect, the present invention provides a kit for screening agonist or inhibitor compound of a protein of interest comprising a nucleic acid or a polynucleotide of the invention, a vector of the invention, a recombinant cell or a host cell of the invention, or a cell-free composition or a composition of the invention, and/or a biosensor of the invention.

The present invention finally provides a method for conducting drug-screening, pharmacologic screening for the identification of new inhibitors/activators of targets, study of molecular pharmacology, discrimination of the effect of a chemical compound/physical stimulus over several pharmacological targets simultaneously, study of the kinetic effect of a chemical compound/physical stimuli on several, simultaneous molecular events and the like, wherein the said method comprises a real-time multiplexed, multi-color BRET assay for determining and\or monitoring various biological activities in live cells, wherein the said assay is capable of capturing multiple signals simultaneously from a single sample and simultaneously over several wells.

The present invention can be used to detect a wide variety of compounds which may act as agonists or antagonists to any proteins of interest.

According to this method of assessing whether a test compound functions as a ligand for a, the method comprising: (i) providing a cell comprising a nucleotide sequence encoding a protein of interest bound to a nucleotide sequence encoding at least one bioluminescent donor molecule and/or bound to a nucleotide sequence encoding at least one acceptor molecule; (ii) contacting said cell with a test compound; and (iii) determining the resultant reaction or interaction and output. The current invention also provides biosensor comprising a nucleotide sequence encoding a voltage-dependent ion channel fusion subunit bound to a nucleotide sequence encoding at least one bioluminescent donor molecule and/or bound to a nucleotide sequence encoding at least one acceptor molecule. Still further the invention provides bioluminescence resonance energy transfer system comprising a nucleotide sequence encoding a protein of interest bound to a nucleotide sequence encoding at least one bioluminescent donor molecule and/or bound to a nucleotide sequence encoding at least one acceptor molecule.

Methods of screening according to the current invention are used for drug discovery and/or development. Also contemplated within the scope of invention is a method of making a pharmaceutical composition comprising (i) performing the method according to the current invention (ii) identifying a test compound that interacts with the protein of interest; and (iii) combining said test compound with a pharmaceutically acceptable carrier.

To that effect the present invention also provides a biosensor or a device for the detection of an analyte that combines a biological component with a physicochemical detector component. It typically consists of three parts, firstly at least one nucleotide molecule encoding the protein of interest. Second, a transducer or detector element, which works in a physicochemical way (e.g. optical, electrochemical) that transforms the signal resulting from the interaction of the compound with the test substance into another signal (i.e. transducers) that can be more easily measured and quantified. Third an associated electronic or signal processor, which then displays the results of the interaction in a user-friendly way.

## EXAMPLES

## Example 1: Multiplexed BRET assays on TRP channels

### Example 1.1: Summary of the Study using multiplexed BRET assay

Multiplexed bioluminescence resonance energy transfer (BRET) assays were developed to monitor the activation of functional TRP (Transient Receptor Potential) channels in live cells in real time. We probed both TRPV1 intramolecular rearrangements and its interaction with Calmodulin under activation by chemical agonists, pH, and temperature. The BRET assay, based on the interaction with Calmodulin, was successfully extended to TRPV3 and TRPV4. A full-spectral three-colour BRET assay capable of analyzing the specific activation of each TRPV channel among the three in a single sample was developed. This major improvement in BRET measurement thus allowed simultaneous monitoring of independent biological pathways in live cells.

TRP proteins form a superfamily of ubiquitously-expressed, functionally-diverse, cation-permeable channels with varying selectivity to several cations. All TRPs are integral proteins containing six transmembrane domains. The N- and C-terminal domains are intracellular and known to be involved in TRP function, regulation, and channel assembly. TRP channels can be activated by several physicochemical means, including the transduction of chemical, temperature, and mechanical stimuli. TRP channels function therefore as polymodal signal integrators that respond by changing their open probability. They are tighly involved into a variety of physiological processes in humans, including sensory physiology, cardiovascular, gastrointestinal, and urological functions, as well as immunity and development. As a result, TRP channel dysfunction has been implicated in many diseases, leading to their emergence as highly promising drug targets. Among the TRP channels, six are recognized as thermo-TRPs, expressed in primary somatosensory neurons and activated at specific temperatures. TRPV1-4 transduce elevated temperatures, ranging from moderate (TRPV3 and TRPV4) to noxious heat (TRPV1 and TRPV2), while TRPM8 and TRPA1 are activated by moderate and extreme cold, respectively.

In this study, we developed intra- and inter-molecular BRET-based biosensors for monitoring the real-time, polymodal activation of TRPV1 channels in live cells. We also extended the intermolecular approach to monitor the chemical activation of both TRPV3 and TRPV4. Finally, using spectral decomposition, we demonstrated the simultaneous monitoring of TRPV1, TRPV3, and TRPV4 ion channel activation in a single assay.

### Example 1.2: Preparation of the plasmid probes

In order to generate the BRET constructs, super Yellow Fluorescent Protein 2 and Renilla Luciferase II were used to improve the brightness of the assay. They are referred as YFP and Luc for short in the rest of the manuscript. The cDNA of Luc and YFP were first cloned together using a three-piece ligation in the BamHI/XhoI site of pcDNA3.1(+) (Invitrogen, Carlsbad, CA, USA), yielding two expression vectors pcDNA3.1 YFP-Luc where YFP was cloned in-fusion at the N-terminal of Luc, and pcDNA3.1 Luc-YFP where Luc was cloned in-fusion at the N-terminal of YFP. Both YFP and Luc were amplified by PCR from the pcDNA3-YFP-EPAC-Luc vector. Luc was cloned either at the N-terminal of YFP as a HindIII-EcoRI fragment (primers used: "Luc_HindIII_ATG_N-term sense" and "Luc_no Stop_EcoRI_N-term antisense") or at the COOH-terminal of YFP as an EcoRI-XhoI fragment (primer used: "Luc_EcoRI_ATG_C-term sense" and "Luc_XhoI_Stop_C-term antisense"). In parallel, YFP was cloned either at the N-terminal of Luc as a HindIII-EcoRI fragment (primers used: "YFP_HindIII_ATG_N-term sense" and "YFP_no Stop_EcoRI_N-term antisense") or at the COOH-terminal of Luc as a EcoRI-XhoI fragment (primer used: "YFP_EcoRI_ATG_C-term sense" and "YFP_XhoI_Stop_C-term antisense"). The sequence joining Luc and YFP sequence encodes VPVNSGGGGS as a linker, and contains an AgeI restriction site.

The YFP-hTRPV1-Luc expression vector was obtained by subcloning the human TRPV1 cDNA from the pDONR201-hTRPV1 vector (Harvard Medical School PlasmID Repository, clone HsCD00081472) as an EcoRI PCR fragment in the EcoRI site of the vector pcDNA3.1 YFP-Luc (primer used: "hTRPV1_EcoRI_Fus_Sense" and "hTRPV1-EcoRI-Fus-antisense"). The hTRPV1-Luc expression vector was obtained by subcloning the cDNA of hTRPV1 as a HindIII-EcoRI fragment in place of the YFP in the HindIII-EcoRI site of the pcDNA3.1 YFP-Luc vector (primer used: "hTRPV1_HindIII_ATG_Sense" and "hTRPV1_EcoRI_Fus_antisense"). The Luc-hTRPV3 expression vector was obtained by subcloning the cDNA of hTRPV3 (Harvard Medical School PlasmID Repository, clone HsCD00341603) as a AgeI-XhoI fragment in place of the YFP in the AgeI-XhoI sites of the pcDNA3.1 Luc-YFP vector (primer used: "hTRPV3_AgeI_ATG_Sense" and "hTRPV3_Stop _XhoI _antisense"). The hTRPV4-Luc expression vector was obtained by subcloning the cDNA of hTRPV4 as a BamHI-AgeI fragment in place of the YFP in the BamHI-AgeI site of the pcDNA3.1 YFP-Luc vector (primer used: "hTRPV4_BamHI_ATG_Sense" and hTRPV4_AgeI_Fus_antisense"). In each case, the sequence joining the cDNA of interest and either Luc or YFP sequence encodes VPVNSGGGGS as a linker.

The cDNA of mAmetrine (Plasmid #54660) and LssmOrange (Plasmid #37130) were obtained from AddGene plasmid repository. The cDNA of aquamarine, mAmetrine and LssmOrange were all subcloned as a HindIII-EcoRI fragment in place of the YFP in the HindIII-EcoRI site of the pcDNA3.1 YFP-Luc vector (primer used: "YFP_HindIII_ATG_N-term sense" and "YFP_no Stop_EcoRI_N-term antisense") to yield pcDNA3.1 FP-Luc expression vectors ("FP" being any fluorescent protein between aquamarine, mAmetrine and LssmOrange). The YFP-CaM expression vector was obtained by subcloning the CaM cDNA in place of the Luc at the C-terminus of YFP into the YFP-Luc as a EcoRI-XhoI fragment (primers used: "hCaM_EcorRI- ATG-Sense" and "hCaM_Stop_Xho_antisense"). A similar strategy was used to obtain the FP-CaM expression vector using the pcDNA3.1 FP-Luc expression vector instead of the pcDNA3.1 YFP-Luc.

**Example 1.3: Primer Selections**

The sequence of each cDNA construct was confirmed by DNA sequencing.

**Example 1.3.1: YFP and Luc primer nucleotide sequences**

Luc_HindIII_ATG_N-term sense,
TGTCTAAGCTTGGATCCGCCACCATGACCAGCAAGGTGTACGACCCCGAGC (SEQ ID NO:1)

Luc_no Stop_EcoRI_N-term antisense,
CACCAGAATTCACCGGTACCTGCTCGTTCTTCAGCACTCTCTCC (SEQ ID NO: 2)

Luc_EcoRI_ATG_C-term sense,
GTGTACCGGTGAATTCTGGTGGAGGCGGATCTATGACCAGCAAGGTGTACGACCCCGAGC (SEQ ID NO: 3)

Luc_XhoI_Stop_C-term antisense,
ATCTAGTCTAGACTCGAGCGGTTACTGCTCGTTCTTCAGCACTCTCTCC (SEQ ID NO: 4)

YFP_HindIII_ATG_N-term sense,
TGTCTAAGCTTGGATCCGCCACCATGGTGAGCAAGGGCGAGGAGCTGTTCACC (SEQ ID NO: 5)

YFP_no Stop_EcoRI_N-term antisense,
CACCAGAATTCACCGGTACCTTGTACAGCTCGTCCATGCCG (SEQ ID NO: 6)

YFP_EcoRI_ATG_C-term sense,
TGTGTACCGGTGAATTCTGGTGGAGGCGGATCTATGGTGAGCAAGGGCGAGGAGCTGTTC (SEQ ID NO: 7)

YFP_XhoI_Stop_C-term antisense,
ATCTAGTCTAGACTCGAGCGGTTACTTGTACAGCTCGTCCATGCCG (SEQ ID NO: 8)

**Example 1.3.2: hTRPV1 primers sequences**

hTRPV1_EcoRI_Fus_Sense,
TGTGTACCGGTGAATTCTGGTGGAGGCGGATCTATGAAGAAATGGAGCAGCACAGACT (SEQ ID NO: 9)

hTRPV1_EcoRI_Fus_antisense,
CACCAGAATTCACCGGTACCTTCTCCCCGGAAGCGGCAGGACTC (SEQ ID NO: 10)

hTRPV1_HindIII_ATG_Sense,
TGTCTAAGCTTGGTACCGCCACCATGAAGAAATGGAGCAGCACAGACT (SEQ ID NO: 11)

**Example 1.3.3: hTRPV3 primers sequences**

hTRPV3_AgeI_ATG_Sense,
TGTCTAAGCTTGGTACCGCCACCATGAAAGCCCACCCCAAGGAGATGG (SEQ ID NO: 12)

hTRPV3_Stop_XhoI_antisense,
ATCTAGTCTAGACTCGAGCGGCTACACCGAGGTTTCCGGGAATTCCTCG (SEQ ID NO: 13)

**Example 1.3.4: hTRPV4 primers sequences**

hTRPV4_BamHI_ATG_Sense
TGTCTGGATCCAAGCTTGCCACCATGGCGGATTCCAGCGAAGGCCCCCG (SEQ ID NO: 14)

hTRPV4_AgeI_Fus_antisense,
CACCAGAATTCACCGGTACGAGCGGGGCGTCATCAGTCCTCCACTTGCG (SEQ ID NO: 15)

**Example 1.3.5: Calmodulin primers sequences**

hCaM_EcoRI_ATG_Sense :

TGTGTACCGGTGAATTCTGGTGGAGGCGGATCTATGGCTGACCAGCTGACTGAGGAGC (SEQ ID NO: 16)

hCaM_Stop_Xho_antisense :

ATCTAGTCTAGACTCGAGCGGTTACTTTGCAGTCATCATCTGTACAAAC (SEQ ID NO: 17)

**Example 1.4: Reagents**

Capsaicin and Capsazepine were all from Tocris (Bristol, UK). Drofenine and GSK1016790A were from Sigma (Lyon, France). AMG517 was from Medchemexpress LLC (Princeton, NJ, USA). Coelenterazine H and Purple Coelenterazine (Nanolight Technology, Pinetop, AZ, USA) were added to a final concentration of 5 $\mu$M.

**Example 1.5: Cell culture and transfections**

HEK293T cells were maintained in Dulbecco's modified Eagle's medium - high Glucose (DMEM) (D6429, Sigma) supplemented with 10 % fetal bovine serum, 100 units mL-1 penicillin and streptomycin. Twenty-four hours before transfection, cells were seeded at a density of 500,000 cells in 6-well dishes. Transient transfections were performed using polyethylenimine (PEI, linear, Mr 25,000; catalogue number 23966 Polysciences, Inc., Warrington, PA, USA) with a PEI:DNA ratio of 4:1. Usually, 0.1-0.25 $\mu$g of the donor constructions and 1.75-1.9 $\mu$g of the acceptors constructions were transfected for the BRET measurement. The amount of transfected DNA was completed to a total of 2 $\mu$g with pcDNA3.1 empty vector. After overnight incubation, transfected cells were then detached, resuspended in DMEM w/o red phenol (Ref 21063-029, ThermoFisher scientific, Waltham, MA, USA) and replated at a density of $10^5$ cells per well in 96-well white plates with clear bottoms (Greiner Bio one, Courtaboeuf, France) pre-treated with D-polylysine (Sigma) for reading with the Tristar2 luminometer (Berthold Technologies, Bad Wildbad, Germany) or onto 12 mm diameter glass coverslips (Knittel Glass, Braunschweig, Germany) treated with poly-L-lysine for the reading with the SpectraPro 2300i spectrometer (Acton Optics, Acton, MA, USA). Cells were left in culture for 24 h before being processed for the BRET assay.

**Example 1.6: Filter-based BRET assays**

For the pharmacological characterization of the probes, agonist and Coelenterazine H were directly added to the cells and BRET assays were performed using a multidetector TriStar2 LB942 microplate reader (Berthold Technologies, Bad Wildbad, Germany) for sequential integration of the signals emitted by all the cell population in each measured well, and detected in the 480 $\pm$ 20 nm and 540 $\pm$ 40 nm bandpass windows for the Luc (energy donor) and the YFP (energy acceptor) light emissions respectively. The BRET signal was determined by calculating the ratio of the emission intensity (I) of the YFP acceptor over that of the Luc donor, according to Eq.1 :

$$(1): \qquad BRET = \frac{I_{YFP}}{I_{Luc}}$$

Due to the overlapping emission spectra of Rluc and EYFP, a fraction of the light detected in the YFP filter originates from the Rluc emission, resulting in a background BRET signal. Using Eq.1, Background BRET signal is similarly measured from cells transfected with the Rluc fusion construct only. Net BRET ratio is calculated by subtracting the background signal detected when the *Luc*-fusion construct was expressed alone, according to Eq.2 :

$$(2): \qquad Net\,BRET = BRET - Background\,BRET$$

To assess the functionality of the probes based on the TRPV channels, agonists and antagonists were added as described in the text for 3 min at given temperature and pH before the addition of Coelenterazine H and BRET reading. All experiments were performed at 37 °C and pH 7.5 unless otherwise indicated.

**Example 1.7 Spectral BRET assays**

Full BRET spectra were acquired using an optical fiber linked to a Spectra Pro 2300i spectrometer, equipped with a liquid-nitrogen-cooled CCD camera for recording the full visible spectrum (Acton Optics, Acton, MA, USA). The bioluminescent signal was recorded from transfected cells seeded onto a glass coverslip and placed into a white opaque measurement chamber made of Teflon® and containing an isotonic solution (NaCl 145 mM, KCl 5 mM, KH2PO4 4 mM, CaCl2 1 mM, MgSO4 1 mM, Glucose 10 mM). The temperature of the cell buffer was regulated using an Eppendorf® ThermoStat Plus and measured in real time using a fiber-optic temperature measurement Luxtron 812 system (Lumasense technologies, Santa Clara, CA, USA).

Using the LabView programming language (National Instruments, Austin, Tx, USA), an interface was developed to run the acquisition of the bioluminescent spectra and perform real-time spectral decomposition of the BRET signal into its various components. The experimental emission spectra of Luc, YFP, mAmetrine, aquamarine, and Lss-mOrange were first obtained experimentally using a Cary Eclipse Fluorimeter (Agilent Technology, Santa Clara, CA, USA) (Figure 3). Each spectrum was then fitted as a sum of Gaussian curves based on Eq.3.:

$$(3): \qquad y = \sum_{i=1}^{n} \Psi_i a_1 e^{\frac{(x-m_i)^2}{2\sigma_i^2}}$$

with $\Psi_n$ being the ratio between $a_i$ and $a_1$, $a_i$ the peak height, mi the wavelength of the peak and σi the width at half-maximum.

The optimized parameters were derived using a standard iterative algorithm based on a non-linear least square fitting method developed by Levenberg & Marquardt. It was then straightforward to calculate the actual BRET ratio for each probe by dividing the area under the acceptor spectrum by that of the donor spectrum. This analysis method represents a major advantage over the standard, filter-based method, for measuring the BRET signal avoiding the contamination of the acceptor signal by the donor emission. The results of the decomposition of the BRET spectra obtained when measuring the bioluminescent signal from a cell population expressing either YFP-Luc, aquamarine-Luc, mAmetrine-Luc, or the Lss-mOrange fusion proteins are given in Figure 4. All experiments were performed at 37 °C unless otherwise indicated.

For experiments under temperature rise, in which the spectral decomposition approach was used along with the SpectraPro 2300i, the area of YFP and Luc emission spectra were corrected for the subtle changes observed between 25 °C and 50 °C (Figure 5). We calculated that YFP emission diminished linearly by 0.46% per °C within the temperature range comprised between 25 °C and 50 °C, while the shape of the spectra was not modified. The YFP emission spectra area was therefore corrected for the temperature-induced variation from 25 °C to 50 °C, according to Eq. 4.:

$$(4): \quad A_{Tcorr} = A_T + A_T(T-25) \times \frac{0.46}{100}$$

with T being any temperature comprised between 25 and 50 °C, $A_T$ being the area of the YFP spectra measured at temperature T and $A_{Tcorr}$ being the corrected area of YFP at the temperature T.

We could calculate that Luc emission spectra shape was slightly modified on its red part inducing a linear increase of 0.17% per degree of its area within the temperature range comprised between 25 °C and 50 °C (Figure 5). The Luc emission spectra area was therefore corrected for the temperature-induced variation from 25 to 50 °C, according to Eq. 5.:

$$B_{Tcorr} = B_T - B_T(T-25) \times \frac{0.17}{100}$$
$$(5):$$

with T being the temperature comprised between 25 and 50 °C, BT the area of the Luc spectrum measured at temperature T and $B_{Tcorr}$ being the corrected area of Luc at temperature T.

**Example 1.8: Calcium assays**

HEK cells were loaded with 0.67 $\mu$M FuraPE3-AM (Teflabs, Austin, USA) for 30 min at 37 °C in Hank' Balanced Salt Solution (HBSS). After washing with PBS, fresh HBSS was added to the cells and calcium measurement was performed at 37 °C using a Flexstation II (Molecular Devices, Sunnyvale, CA, USA). Fura2-AM was alternately excited at 340 and 380 nm and emission was read at 510 nm. The 340/380 nm ratio was used to estimate the variations of cytosolic calcium concentration.

**Example 1.9: Data analysis**

Data obtained in BRET and calcium assays were analysed using the Prism 6.01 software (GraphPad Software, Inc, La Jolla, CA, USA).

**Example 2: Validation of the TRPV1 BRET biosensors**

Knowing that: (i) FRET analysis revealed that movements within intracellular regions of the TRP-structurally related Kv2.1 channel were part of the gating machinery and (ii) TRPV1 contains Calmodulin (CaM) binding sequences, we hypothesized that BRET could be used to monitor TRP channel activation in live cells. TRPV1, the most studied channel in the TRP family, was therefore used as our main model for probing channel conformational changes and CaM docking following activation. For this purpose, we developed two proximity-based BRET assays, relying on the energy donor Renilla reniformis luciferase (Luc), fused to the C-terminus part of the TRPV1 protein, and the energy acceptor Yellow Fluorescent Protein (YFP), fused to the N-terminus part of either TRPV1-Luc or CaM. In the first case, the TRPV1 protein was sandwiched between the Luc and YFP groups, resulting in an intramolecular BRET probe, referred to below as YFP-TRPV1-Luc , while, in the second case, YFP-CaM docking on TRPV1-Luc (TRPV1-Luc/YFP-CaM) was monitored.

TRP channel activation was evaluated in transfected HEK293T human embryonic-kidney cells. We first assessed whether, following transfection, our TRPV1-fusion proteins remained functional despite the N-and/or C-terminus addition of the YFP or Luc groups. For this purpose, we measured calcium entry in mock-transfected or transfected HEK293T cells with either native TRPV1 or the BRET constructs, YFP-TRPV1-Luc or TRPV1-Luc. Following exposure to Capsaicin (CAPS), the prototypical TRPV1 agonist, a rapid, maintained increase in cytosolic calcium concentration was observed in cells expressing TRPV1, YFP-TRPV1-Luc, or TRPV1-Luc, but not in mock-transfected cells. This indicated that the addition of either the YFP and/or Luc groups did not hinder TRPV1 channel opening, in agreement with previous data. HEK293T cells expressing YFP-TRPV1-Luc or TRPV1-Luc/YFP-CaM were then processed for BRET analysis. In both cases, a basal BRET signal was observed that was increased with a first order kinetic following exposure to capsaicin.

The increase of the basal BRET signal measured from the intramolecular TRPV1 BRET probe may perfectly reflect the changes in the different modes of energy transfer between Luc and YFP inside the TRPV1 tetrameric organization of the channel during channel opening. However, the increase of the basal BRET between TRPV1 and Calmodulin was more elusive since it could result either from a conformational change in a pre-assembled TRPV1-CaM complexe and/or from a modification of the association-dissociation equilibrium between these two partners. Interestingly, both chelation of the intracytoplasmic calcium pool using BAPTA-AM or the use as an acceptor of a YFP-tagged CaM mutant, with all four calcium binding sites mutated (YFP-CaM$_{1234}$), severely diminished the basal BRET signal and abolished CAPS-induced BRET increase. These results indicate that TRPV1 and a pool of calcium-bound CaM could be pre-associated in resting state and that calcium-bound CaM is mandatory to observe the CAPS-induced BRET increase in our TRPV1-Luc/YFP-CaM intermolecular BRET assay. To further characterize the interaction between TRPV1 and CaM, we performed Full BRET titration curves of the TRPV1-Luc / YFP-CaM BRET pair in the presence and absence of CAPS. In absence of CAPS the BRET signal increased as a hyperbolic function of the YFP-CaM / TRPV1-Luc ratio indicative of a specific protein-protein interaction (Mercier, 2001; percherancier, 2005). The selectivity of the measured signal is further supported by the fact that co-expression of TRPV1-Luc with YFP-CaM$_{1234}$ led to a weaker signal that progressed linearly over the same range of energy acceptor/donor. Since random molecular collisions that would give rise to bystander BRET have been shown to increase nearly linearly over a wide range of YFP/RLuc (Mercier, 2001; percherancier, 2005, hamdan 2006), this last result definitely indicates that TRPV1 cannot engage into an interaction with calcium-free CaM. The addition of CAPS dramatically increased the maximal BRET signal observed and slightly but significantly affected the shape of the curve so that the concentration of YFP-CaM needed to reach 50% of the maximal BRET signal (BRET$_{50}$) was diminished (values to indicate). Because the BRET$_{50}$ represents the propensity of the BRET partners to interact with one another (*i.e.* their relative affinity), our data indicate that the CAPS treatments did increase the number of TRPV1 / CaM complexes. The maximal BRET signal increase indicating that conformational changes within preformed TRPV1 / CaM complexes affect the distance between the energy donor and acceptor. Finally, ionomycin had no effect on the TRPV1/CaM basal BRET signal, suggesting that TRPV1 needs to be activated first to engage more Calcium-bound CaM at a later time.

We next confirmed that the agonist-induced increase in the BRET signal was dose dependent with half-maximal responses (Table 1) consistent with those reported in the literature, using patch-clamp or calcium-flux measurements on cells expressing endogenous or over-expressed TRPV1. The pharmacological selectivity of the ligand-promoted BRET changes was further demonstrated by the competitive nature of the effects, as both Capsazepine (CPZ) and AMG517, two well-known competitive TRPV1 antagonists, inhibited the CAPS-induced BRET increase, as illustrated by the shift in CAPS potency to higher values in both intra- and inter-molecular BRET tests (Table 1). Altogether, these data strongly suggested that the agonist-promoted BRET changes in both probe configurations corresponded to an activation of TRPV1 channels in live cells.

**Table 1:** CAPS potency derived from BRET assays carried out in HEK293T expressing either YFP-TRPV1-Luc or TRPV1-Luc/YFP-CaM, activated with increasing doses of CAPS, with or without inhibitors (vehicle, CPZ 1$\mu$M, or AMG517 1 $\mu$M). BRET assays were analyzed by nonlinear regression using the GraphPad-Prism software. Potency, expressed as Log EC$_{50}$ (M), was derived from sigmoidal dose-response curve fitting. Values represent the mean $\pm$ standard error of four independent experiments performed in duplicate. Asterisks indicate statistical significance of the difference between the inhibitors conditions and control condition (CAPS alone) with ****, $p < 0.0001$; ***, $p < 0.001$; **, $p < 0.01$. "ns" indicates $p > 0.05$.

Table 1:

| | Inhibitors | | | |
| --- | --- | --- | --- | --- |
| | None (CAPS alone) | Vehicle | AMG517 | CPZ |
| YFP-TRPV1-Luc | $-6.50 \pm 0.11$ | $-6.44 \pm 0.11^{ns}$ | $-5.07 \pm 0.10^{***}$ | $-4.57 \pm 0.16^{**}$ |
| TRPV1-Luc/YFP-CaM | $-6.53 \pm 0.16$ | $-6.64 \pm 0.14^{ns}$ | $-4.78 \pm 0.09^{****}$ | $-4.29 \pm 0.19^{**}$ |

**Example 3: Heat activation of TRPV1 monitored by BRET**

Another series of experiments assessed the effect of temperature on our BRET probes. The BRET signal was measured in real time while heating the cell culture from 25 to 50 °C. In cells expressing the TRPV1-Luc/YFP-CaM construct, the initial basal BRET signal remained stable between 25 and 37 °C. It then increased between 37 and 45 °C before reaching a plateau. When the same assay was repeated in a calcium-free buffer or when the cells were preincubated with CPZ, there was no significant increase in the BRET signal, indicating that the increase observed was fully related to temperature-dependent channel activation and calcium entry through the channel. In cells expressing YFP-TRPV1-Luc, the BRET signal remained stable up to 37 °C, then decreased dramatically up to 50 °C. Preincubation with CPZ before heating completely modified the temperature-dependent behaviour of the YFP-TRPV1-Luc probe: the basal BRET was stable up to 37 °C but the signal increased dramatically from 37 to 47 °C, before decreasing sharply up to 50 °C. Considering that CPZ blocks the opening of the channel over this temperature range, these results indicated that TRPV1 underwent some complex temperature-dependent conformational changes despite the channel remaining in the closed state.

Since it has been proposed that the various TRPV1 activation modes are independently coupled to channel gating, we investigated whether heating sensitized TRPV1 to CAPS. Using both probes, a temperature rise from 25 to 42 °C resulted in both a leftward shift in CAPS potency and an increase in maximal efficacy. This observation was in agreement with previous studies using the patch-clamp technique and indicated that the temperature response of both YFP-TRPV1-Luc and TRPV1-Luc/YFP-CaM mimicked that of the native TRPV1 channel. Both intra- and inter-molecular probes were thus equally well-suited to monitoring TRPV1 channel activity. Since TRP channels are known to engage a large network of protein-protein interactions under resting or activated conditions, the inter-molecular-based approach is more promising for studies on CaM-interacting channels or other signaling pathways, while the intramolecular probe is more appropriate for structure-function studies.

## Example 4: Extending the CaM interaction BRET test to TRPV3 and TRPV4 monitoring

The final aim of this work was to monitor the concomitant activation of up to three TRP channels. Consequently, knowing that TRPV3 and TRPV4 also interact with CaM (22), we monitored the activation of TRPV3 and TRPV4 by their specific agonists using the CaM BRET probe approach. As shown in Figure 1, a dose-dependent increase in BRET signal between YFP-CaM and Luc-TRPV3 was observed when the transfected cells were challenged with Drofenine, a specific TRPV3 agonist, yielding an $EC_{50}$ of 206 $\mu$M in agreement with the literature. We found no dose-dependent BRET increase when the same cells were challenged with either the TRPV1-specific agonist CAPS or the TRPV4-specific agonist GSK1016790A. Similarly, a dose-dependent increase in the TRPV4-Luc/YFP-CaM basal BRET signal was observed when the transfected cells were challenged with GSK1016790A, yielding an $EC_{50}$ of 1.93 nM, also in agreement with the literature. As shown for TRPV3, cross activation of TRPV4-Luc/YFP-CaM-expressing cells with either CAPS or Drofenine did not produce any increase in the basal BRET signal. We also showed that TRPV1-Luc/YFP-CaM interaction was sensitive to CAPS, but not to Drofenine or GSK1016790A (Figure 1). These results demonstrated the effectiveness and flexibility of the CaM-binding BRET test for monitoring the chemical activation of CaM-interacting TRP channels.

## Example 5: Multiplexed BRET monitoring of three TRPV channels using spectral decomposition

We then designed a single test to monitor the activity of several TRP channels simultaneously. The standard filter-based BRET approach constituted a technological barrier to performing a multi-colour BRET assay with more than two colours. We therefore developed for three acceptors the full-spectral analysis of BRET signals, that we already implemented for one acceptor. For this purpose, we used a blue-shifted luciferase for better separation of acceptors, as can be obtained with the BRET2 configuration. However, since the advantages associated with the BRET2 system were partly offset by the low quantum yield and the rapid decay kinetics of the coelenterazine-400a donor substrate, we used the recently-developed methoxy e-Coelenterazine, also known as "purple coelenterazine" that yields up to 13 times more luminescence, with a maximal emission at 425 nm. Moreover, Luc2 was used as a donor, as this RLuc mutant yields luminescence signals 50 times brighter than those generated by WT Rluc.

It is known that aquamarine, mAmetrine, and LSSmOrange are all fluorescent proteins, excitable in the 400-430 nm range, with sufficiently different Stoke's shifts to provide good separation of their emission spectra. We verified experimentally that the bioluminescence emission spectrum of the Luc enzyme in the presence of purple coelenterazine substrate matched the excitation spectra of aquamarine, mAmetrine, and LSSmOrange (Figure 3). As previously done for YFP, we modelled the shape of the spectra of aquamarine, mAmetrine, and LSSmOrange and implemented them in our spectral BRET analysis (see material and methods). The HEK293T cell populations expressing aquamarine-Luc, mAmetrine-Luc, or LSSmOrange-Luc fusion proteins were then mixed in one dish and the multicomponent BRET spectrum was acquired. Our algorithm fitted a theoretical function to the experimental data obtained, which intrinsically contained the functions of all components (Figure 2A). It was then straightforward to calculate the BRET ratio for each probe by dividing the area of the respective spectrum by that of the donor spectrum. We then mixed together HEK293T cells expressing Luc-TRPV3/aquamarine-CaM, TRPV1-Luc/mAmetrine-CaM and TRPV4-Luc/LSSmOrange-CaM and performed the spectral decomposition of the complex multicolour BRET signal, during agonist activation, in real time. As shown in Figure 2B, this three-color spectral BRET analysis revealed the selective activation of each TRPV channel in a single well. In each case, agonist stimulation induced a specific, increase in its cognate TRPV BRET probe signal. Drofenine injection induced an increase in only the TRPV3-related BRET component (Figure 1B), while activation with CAPS or GSK1016790A induced a time-dependent increase in only the TRPV1 or TRPV4 BRET signals, respectively (Figure 2 C and D).

**Example 6: Results**

We report here the characterization of probes for real-time BRET measurement of TRPV1, TRPV3, and TRPV4 ion-channel activation in live cells. A decomposition of the whole emission spectrum of the BRET signal, instead of the usual selective filter-based approach, provided for the first time a reliable method for performing three-color BRET tests. This novel approach was used to observe the selective activation of TRPV1, TRPV3, and TRPV4 in a single assay, simultaneously, in real time.

The Fluorescence Resonance Energy Transfer (FRET) technique, based on intra- and inter-molecular probes, has previously been used to probe conformational changes in various channels in live cells or membranes during activation. Alternatively, FRET has also been applied to studying the interactions of some ion channels with partners, such as Calmodulin. These assays offer the advantage of single-cell microscopy imaging that may be combined with voltage-clamp conditions, thus providing a precise control of channel activation while recording the FRET signal. Eliminating the need for an external light source for donor excitation gives BRET some advantages over FRET: it does not cause photodamage to cells, photobleaching of fluorophores, background auto fluorescence, or direct excitation of the acceptor. Thanks to these advantages, the BRET technique has been widely implemented for drug screening, especially in the GPCR research field. Implementation of high-throughput screening (HTS) on ion channels, including TRPs, has proved more problematic. The gold standard for evaluating the activity of TRPs and other ion channels is patch-clamp electrophysiology. Improvements that increase throughput for the direct screening of ion channel targets are rapidly emerging, including automated electrophysiology and planar patch-clamp techniques. These approaches remain expensive and require expert handling. For HTS, indirect readout technologies are often used as an initial screening step, later confirmed by patch-clamp. These techniques usually rely on fluorescent assays to monitor changes in membrane potential or intracytoplasmic calcium concentrations. Nonetheless, indirect assays of ion channel function often produce false-positive hits, as they monitor endpoints distal from the channel, separated by multiple steps in the signalling pathways. Measuring events proximal to receptor activation reduces the probability of false positives. Therefore, the advent of BRET probes for monitoring the activation of channels in live cells in real time is most valuable.

While steady-state TRPV1 subunit oligomerization had been previously studied using either FRET, or a combination of BiMolecular Fluorescence complementation and BRET, none of these authors could show any variation of the measured signal following TRPV1 activation. Several studies succeeded in measuring ion-channel activation using intra- or intermolecular FRET based probes, but only one research group successfully reported the use of BRET-based biosensors to monitor ion-channel activity, focusing on the Kir3 inwardly-rectifying potassium channel in combination with FlAsH (fluorescein arsenical hairpin binder). Adapting the FlAsH/BRET approach to other channels would require extensive studies to determine how to insert the FlAsH sequence into the channel structure to yield optimal variation in BRET signal upon channel activation. Moreover, the alterations in BRET signal reported by Robertson et al. (2016) were often weak, not exceeding 5-10% of the basal BRET signal. In sharp contrast, our experiments using TRPV-Luc/YFP-CaM detected significantly larger increases upon activation, ranging from 65 to 115 % of the corresponding basal net BRET (Figure 2). These probes offer a wide potential for developing simple cell-based assays that provide direct information on channel activity, especially in drug screening.

Our study, moreover, provided new light on the interaction between CaM and TRPV1. Calmodulin has been identified as a component of the TRPV1 inactivation machinery, although discrepancies have existed as its Ca2+ dependence for TRPV1 interaction as well as its binding site on TRPV1. Using classical disruptive biochemical techniques showed that $Ca^{2+}$-bound CaM but not $Ca^{2+}$-free CaM binds to one of the unconventional TRPV1 channel CaM binding sites while Rosenbaum et al.(2014) showed that the fraction of CaM bound to TRPV1 is unchanged in the presence or absence of Ca2+. Using our TRPV1-Luc / YFP-CaM intermolecular BRET assay, we could assess that TRPV1 and Ca2+-bound CaM are pre-associated in resting living cells. Our results also confirm the earlier observations showing that more TRPV1-CaM complexes are formed upon CAPS activation. BRET titration curves clearly indicate that no specific interaction could be measured between TRPV1 and Ca2+-free CaM even after CAPS activation. Finally, our results indicate that conformational change do occurs during CAPS activation of TRPV1 that impact the orientation and or the distance between Luc on TRPV1-Luc and YFP on YFP-CaM proteins, leading to a higher maximal BRET.

In these experiments, the intramolecular BRET probe was not used to investigate TRPs other than TRPV1, but represents a promising tool for elucidating TRPV1 gating. We observed that, while CAPS treatment induced an increase in the basal BRET signal of YFP-TRPV1-Luc, heating produced multiple conformational changes. In agreement with a previous study, this indicated that CAPS and heat triggered distinct conformational transitions, both resulting in channel-pore opening. these results indicated that TRPV1 underwent these results indicated that TRPV1 underwent these results indicated that TRPV1 underwent.

A voltage-sensitive mechanism has been initially proposed to underlie gating of thermo-sensitive TRP channels. According to this hypothesis, TRP channels are intrinsically voltage sensitive and thermal and chemical stimuli act to increase this voltage sensitivity. Nonetheless, an allosteric model in which voltage, temperature, agonists and inverse agonists are independently coupled, either positively or negatively, has been proven to be more accurate in describing many aspects of TRPV1 gating. We also observed that heat increases CAPS potency and efficacy using both intra- and intermolecular BRET test. Altogether, our results are in full agreement with the findings that capsaicin and heat promote distinct transitions that are allosterically coupled during channel pore gating.

The fact that a simple BRET assay was capable of monitoring the chemical activation of three TRPV channels, one at a time, prompted us to measure TRPV1, TRPV3, and TRPV4 activity in a single assay. One of the greatest obstacles to achieving quantitative multiplexed BRET measurements is the overlap among donor and acceptors emission spectra. One approach to by-pass this technical barrier, using a two-color BRET assay, is to fine-tune filter sets for sequential measurement of the energy transfer between Luc and two fluorescent acceptors with sufficiently separated emission spectra. These authors made a trade-off between the amount of cross-contamination of each acceptor considered, using various filter sets, and the transfer efficiency between Luc and the acceptors. In order to overcome the limitations of the filter-based strategy, we performed a full spectral decomposition of the BRET signal. Using full-spectral multiplexing, it thus became possible to assess the selective activation of TRPV1, TRPV3, and TRPV4 channels in a single sample. This provided perspectives for evaluating the specificity of particular drugs for TRPV subtypes from a single experiment.

Full-spectral BRET multiplexing may also be of importance to monitor several molecular events simultaneously or to evaluate the kinetic of their engagement. It is known that a single receptor in the G-protein coupled receptor family engages different signaling pathways and that various drugs binding to this membrane protein may differentially influence each of them, leading to a reassessment of the efficacy concept. In other words, ligands that are agonist for a given signaling pathway may act as antagonist or even inverse agonist for a different pathway via the same receptor. Whether this concept is also applicable to voltage-gated channels, especially TRPs, remains to be determined. The large network of protein-protein interactions in ion-channel pathways offers a rich source of potential drug targets. The TRPV1 channel, for example, has been shown to interact with multiple partners, such as Caveolin, β-Arrestin-2, AKAP79/150, and PKCβ2, as well as other TRP channels. Constructing BRET probes to test the interactions between TRPV1 and each of these partners will greatly contribute to resolving the complex, dynamic interplay between TRPV1 and its interactome, thus offering new effective methods for screening macromolecular complexes in search of new compounds that target protein-channel interfaces. In this context, monitoring multiple signaling pathways via a single multi-color assay protocol represents a highly valuable development.

This report describes an efficient technique for collecting three BRET signals simultaneously from one sample. Instead of using one optical fiber to collect the photons from a sample, it is technically possible to use a bundle of many optical fibers to collect the BRET spectra from multiple samples in real time. This simultaneous recording of the dynamics of three BRET probes in many samples in parallel is likely to provide highly valuable data for drug screening. Channel-specific BRET probes for TRPV1/3/4 will lead to multi-BRET probe readings in a multi-well format, which undoubtedly represents a breakthrough in ion-channel drug screening and drug discovery in general.

**Claims**

1. A real-time multiplexed, multi-color bioluminescence resonance energy transfer (BRET) technology-based assay for detecting and\or monitoring one or more proteins-proteins interactions simultaneously in live cells and optionally in one or multiple reaction wells,

   wherein said live cells are recombinant cells comprising one or more molecular probes carrying bioluminescent donor molecules, and one or more molecular probes carrying at least two fluorescent acceptor molecules,

   wherein said bioluminescent donor and its corresponding fluorescent acceptor molecules form donor-acceptor couple which are selected such that the emission spectrum of the bioluminescent donor molecule overlaps with the absorbance spectrum of the fluorescent acceptors molecules, thereby (i) generating transfers of energy in parallel from more than one molecular probe carrying bioluminescent donor molecule to more than one molecular probe counterpart fluorescent acceptor molecules, or (ii) generating transfers of energy from one molecular probe carrying a bioluminescent donor molecule to more than one molecular probe carrying counterpart fluorescent acceptor molecules, or (iii) generating transfers of energy from one molecular probe carrying bioluminescent donor molecule to at least one molecular probe carrying fluorescent acceptor molecule, whereby the resulting emission spectrum of said activated fluorescent acceptor molecule overlaps with the absorbance spectrum of a subsequent acceptor molecule, thereby allowing transfer of energy in cascade of subsequent fluorescent acceptor molecules,

   wherein energy signals of said each donor-acceptor couples are sufficiently distinct so as to allow spectral decomposition,

   said assay comprising the steps of:

   (i) contacting live recombinant cells with an activation or inhibition signal;
   (ii) capturing multiple energy signals from each donor-acceptor couple simultaneously from a single sample and simultaneously over one or several wells,
   (iv) processing said multiple energy signals by spectral decomposition.

2. Real-time multiplexed, multi-color BRET assay according to claim 1 wherein multiple energy signals are captured across visible spectra close to or within the infrared spectrum.

3. Real-time multiplexed, multi-color BRET assay according to claim 1 or 2 wherein the said spectra is between 400 to 800 nm.

4. Real-time multiplexed, multi-color BRET assay according to any one of the preceding claims, wherein the said assay enables the measurement of the BRET signals in a single reading and one output.

5. Real-time multiplexed, multi-color BRET assay according to any one of the preceding claims, wherein the said assay is not dependent on selective filter-based approach.

6. Real-time multiplexed, multi-color BRET assay according to any one of the preceding claims, wherein said assay is based on full spectral multi-color output.

7. Real-time multiplexed, multi-color BRET assay according to any one of the preceding claims, wherein said bioluminescent protein is a receptor or a voltage-dependent ion channel.

8. Real-time multiplexed, multi-color BRET assay according to any one of the preceding claims , wherein said one or more bioluminescent donor molecules and fluorescent acceptor molecules are fused to proteins of interest within said molecular probes, thereby allowing monitoring and/or detection of said proteins-proteins interactions.

9. Real-time multiplexed, multi-color BRET assay according to claim 8 wherein said protein of interest is a membrane protein, a cytoplasmic protein, a nuclear protein.

10. Real-time multiplexed, multi-color BRET assay according to any one of the preceding claims, wherein said bioluminescent molecule is a protein chosen from among luciferase, chosen among Renilla luciferase, Firefly luciferase, Coelenterate luciferase, North American glow worm luciferase, click beetle luciferase, a railroad worm luciferase, Gaussia luciferase, Aequorin, Arachnocampa luciferase, or a biologically active variant or fragment of any one, or non-luciferase bioluminescent protein chosen among β-galactosidase, lactamase, horseradish peroxydase, alkaline phosphatase, β-glucuronidase, or β-glucosidase.

11. Real-time multiplexed, multi-color BRET assay according to any one of the preceding claims, wherein said fluorescent molecule is a protein chosen from among green fluorescent protein (GFP), variant of green fluorescent protein (GFP10), blue fluorescent protein (BFP), cyan fluorescent protein (CFP), yellow fluorescent protein (YFP), enhanced GFP (EGFP), enhanced CFP (ECFP), enhanced YFP (EYFP), GFPS65T, Emerald, Topaz, GFPuv, destabilised EGFP (dEGFP), destabilised ECFP (dECFP), destabilised EYFP (dEYFP), HcRed, t-HcRed, DsRed, DsRed2, mRFP1, pocilloporin, Renilla GFP, Monster GFP, paGFP, Kaede protein or a Phycobiliprotein, or a biologically active variant or fragment of any one thereof, or wherein the acceptor molecule is Alexa, fluor dye, Bodipy dye, Cy dye, fluorescein, dansyl, umbelliferone, fluorescent microsphere, luminescent nanocrystal, Marina blue, Cascade blue, Cascade yellow, Pacific blue, Oregon green, Tetramethylrhodamine, Rhodamine, Texas red, rare earth element chelates, mAmetrine, LSSmOrange, aquamarine or any combination or derivatives thereof.

12. Apparatus for performing real-time multiplexed, multi-color BRET assay according to any one of claims 1-11, comprising a real-time BRET instrument and multiple reaction wells for containing said live cells, wherein the said BRET instrument comprises a spectrophotometer with a suitable imaging system, one or more optic fibers, said spectrophotometer with suitable imaging system and optic fibers being connected to a computer equipped with an information interface for the collection and interpretation of the decomposition of the spectral signals acquired and/or for sending back the form and area of the spectra of the energy donor and of the energy acceptors in a quantitative manner.

13. Apparatus of claim 12, wherein spectrophotometer with a suitable imaging system comprises features selected from one or more of diffraction grating, hyper spectral imaging, and/or a CCD camera.

14. A method of drug-screening or pharmacologic screening for the identification of new inhibitors or activators of protein targets, discrimination of the effect of a chemical compound/physical stimulus over several pharmacological targets simultaneously, study of the kinetic effect of a chemical compound/physical stimuli on several, simultaneous molecular events, said method comprising contacting said live cells as defined in claim 1 with a chemical compound or physical stimulus, providing a substrate of the bioluminescent donor molecule, proceeding to the spectral decomposition of the multiple energy signals obtained.

**Figure 1**

# Figure 2

Figure 3

Figure 4

A

B

# Figure 5

A

B

C

D

Europäisches
Patentamt

European
Patent Office

Office européen
des brevets

# EUROPEAN SEARCH REPORT

Application Number

EP 16 19 2290

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2016/131832 A1 (UNIV DE BORDEAUX [FR]; CENTRE NAT RECH SCIENT [FR]; INST POLYTECHNIQUE) 25 August 2016 (2016-08-25) * the whole document * | 1-14 | INV.<br>G01N33/533<br>G01N33/58<br>G01N33/542 |
| Y | WO 2013/155553 A1 (COMMW SCIENT IND RES ORG [AU]) 24 October 2013 (2013-10-24) * claims * * page 26, line 21 - line 23 * * figures 31,47 * | 1-14 | |
| Y | WO 2005/050182 A1 (UNIV WESTERN AUSTRALIA [AU]; SCHMIDT ULRICH [AU]; EIDNE KAREN [AU]; KR) 2 June 2005 (2005-06-02) * claims * * paragraph [0009] - paragraph [0012] * * paragraph [0030] - paragraph [0032]; figures 1-3 * * paragraph [0100] * * example 2 * | 1-14 | |
| Y | US 2005/059031 A1 (BRUCHEZ MARCEL P [US] ET AL) 17 March 2005 (2005-03-17) * page 28, right-hand column, paragraph 296 - paragraph 297 * | 1-14 | TECHNICAL FIELDS SEARCHED (IPC)<br><br>G01N |
| Y | WO 2004/102165 A1 (NOVASITE PHARMACEUTICALS INC [US]; BEERNINK ANDREW [US]; BENNETT TERES) 25 November 2004 (2004-11-25) * claims * * page 10, line 1 - line 9 * | 1-14 | |
| Y | US 6 337 472 B1 (GARNER HAROLD R [US] ET AL) 8 January 2002 (2002-01-08) * claims * * column 5, line 39 - line 50 * | 1-14 | |

-/--

The present search report has been drawn up for all claims

| Place of search | Date of completion of the search | Examiner |
|---|---|---|
| The Hague | 20 March 2017 | Routledge, Brian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 16 19 2290

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 5 828 452 A (GILLISPIE GREGORY [US] ET AL) 27 October 1998 (1998-10-27)<br>* column 1, line 44 - line 46 *<br>* column 4, line 17 - line 31 *<br>----- | 1-14 | |
| | | | **TECHNICAL FIELDS SEARCHED (IPC)** |

The present search report has been drawn up for all claims

| Place of search | Date of completion of the search | Examiner |
|---|---|---|
| The Hague | 20 March 2017 | Routledge, Brian |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 16 19 2290

This annex lists the patent family members relating to the patent documents cited in the above-mentioned European search report.
The members are as contained in the European Patent Office EDP file on
The European Patent Office is in no way liable for these particulars which are merely given for the purpose of information.

20-03-2017

| Patent document cited in search report | Publication date | Patent family member(s) | Publication date |
|---|---|---|---|
| WO 2016131832 A1 | 25-08-2016 | EP 3056902 A1<br>WO 2016131832 A1 | 17-08-2016<br>25-08-2016 |
| WO 2013155553 A1 | 24-10-2013 | AU 2013204332 A1<br>CA 2869914 A1<br>CN 104380085 A<br>EP 2839266 A1<br>HK 1207421 A1<br>JP 2015521274 A<br>KR 20150005619 A<br>NZ 630059 A<br>SG 11201405952U A<br>US 2015094219 A1<br>WO 2013155553 A1 | 31-10-2013<br>24-10-2013<br>25-02-2015<br>25-02-2015<br>29-01-2016<br>27-07-2015<br>14-01-2015<br>24-02-2017<br>30-10-2014<br>02-04-2015<br>24-10-2013 |
| WO 2005050182 A1 | 02-06-2005 | CA 2546764 A1<br>EP 1687615 A1<br>JP 2007511226 A<br>US 2008108128 A1<br>WO 2005050182 A1 | 02-06-2005<br>09-08-2006<br>10-05-2007<br>08-05-2008<br>02-06-2005 |
| US 2005059031 A1 | 17-03-2005 | CA 2550151 A1<br>EP 1700123 A2<br>JP 2007521332 A<br>US 2005059031 A1<br>US 2011136139 A1<br>US 2015198606 A1<br>WO 2005081721 A2 | 09-09-2005<br>13-09-2006<br>02-08-2007<br>17-03-2005<br>09-06-2011<br>16-07-2015<br>09-09-2005 |
| WO 2004102165 A1 | 25-11-2004 | AU 2004239716 A1<br>CA 2524782 A1<br>EP 1625385 A1<br>JP 2007504837 A<br>US 2005009060 A1<br>WO 2004102165 A1 | 25-11-2004<br>25-11-2004<br>15-02-2006<br>08-03-2007<br>13-01-2005<br>25-11-2004 |
| US 6337472 B1 | 08-01-2002 | NONE | |
| US 5828452 A | 27-10-1998 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016131832 A

- US 6949377 B

**Non-patent literature cited in the description**

- **BRETON B. et al.** *Biophysical Journal,* December 2010, vol. 99, 4037-4046

- **SPIBEY et al.** *Electrophoresis,* 2001, vol. 22, 829-836

SEQUENCE LISTING

<110>  UNIVERSITE DE BORDEAUX
       CENTRE NATIONAL DE LA RECHERCHE SCIENTIFIQUE
       INSTITUT POLYTECHNIQUE DE BORDEAUX

<120>  NOVEL REAL-TIME MULTIPLEXED, MULTI-COLOR BIOLUMINESCENCE
       RESONANCE ENERGY TRANSFER ASSAY, APPARATUS, AND USES THEREOF

<130>  1610-EPO

<140>  Not Yet Known
<141>  2016-10-04

<160>  17

<170>  PatentIn version 3.5

<210>  1
<211>  51
<212>  DNA
<213>  Artificial Sequence

<220>
<223>  primer

<400>  1
tgtctaagct tggatccgcc accatgacca gcaaggtgta cgaccccgag c                     51


<210>  2
<211>  44
<212>  DNA
<213>  Artificial Sequence

<220>
<223>  primer

<400>  2
caccagaatt caccggtacc tgctcgttct tcagcactct ctcc                             44


<210>  3
<211>  60
<212>  DNA
<213>  Artificial Sequence

<220>
<223>  primer

<400>  3
gtgtaccggt gaattctggt ggaggcggat ctatgaccag caaggtgtac gaccccgagc           60


<210>  4
<211>  49
<212>  DNA
<213>  Artificial Sequence

<220>
<223>  primer

<400>  4

atctagtcta gactcgagcg gttactgctc gttcttcagc actctctcc                    49


<210>    5
<211>    53
<212>    DNA
<213>    Artificial Sequence

<220>
<223>    primer

<400>    5
tgtctaagct tggatccgcc accatggtga gcaagggcga ggagctgttc acc              53


<210>    6
<211>    41
<212>    DNA
<213>    Artificial Sequence

<220>
<223>    primer

<400>    6
caccagaatt caccggtacc ttgtacagct cgtccatgcc g                           41


<210>    7
<211>    60
<212>    DNA
<213>    Artificial Sequence

<220>
<223>    primer

<400>    7
tgtgtaccgg tgaattctgg tggaggcgga tctatggtga gcaagggcga ggagctgttc       60


<210>    8
<211>    46
<212>    DNA
<213>    Artificial Sequence

<220>
<223>    primer

<400>    8
atctagtcta gactcgagcg gttacttgta cagctcgtcc atgccg                      46


<210>    9
<211>    58
<212>    DNA
<213>    Artificial Sequence

<220>
<223>    primer

<400>    9
tgtgtaccgg tgaattctgg tggaggcgga tctatgaaga aatggagcag cacagact         58

```
<210>   10
<211>   44
<212>   DNA
<213>   Artificial Sequence

<220>
<223>   primer

<400>   10
caccagaatt caccggtacc ttctccccgg aagcggcagg actc                        44


<210>   11
<211>   48
<212>   DNA
<213>   Artificial Sequence

<220>
<223>   primer

<400>   11
tgtctaagct tggtaccgcc accatgaaga aatggagcag cacagact                    48


<210>   12
<211>   48
<212>   DNA
<213>   Artificial Sequence

<220>
<223>   primer

<400>   12
tgtctaagct tggtaccgcc accatgaaag cccaccccaa ggagatgg                     48


<210>   13
<211>   49
<212>   DNA
<213>   Artificial Sequence

<220>
<223>   primer

<400>   13
atctagtcta gactcgagcg gctacaccga ggtttccggg aattcctcg                   49


<210>   14
<211>   49
<212>   DNA
<213>   Artificial Sequence

<220>
<223>   primer

<400>   14
tgtctggatc caagcttgcc accatggcgg attccagcga aggcccccg                   49


<210>   15
<211>   49
<212>   DNA
```

```
<213>  Artificial Sequence

<220>
<223>  primer

<400>  15
caccagaatt caccggtacg agcggggcgt catcagtcct ccacttgcg                    49


<210>  16
<211>  58
<212>  DNA
<213>  Artificial Sequence

<220>
<223>  primer

<400>  16
tgtgtaccgg tgaattctgg tggaggcgga tctatggctg accagctgac tgaggagc         58


<210>  17
<211>  49
<212>  DNA
<213>  Artificial Sequence

<220>
<223>  primer

<400>  17
atctagtcta gactcgagcg gttactttgc agtcatcatc tgtacaaac                   49
```